(19) Europäisches Patentamt
European Patent Office
Office européen des brevets

(11) Veröffentlichungsnummer: **0 277 088 A2**

# (12) EUROPÄISCHE PATENTANMELDUNG

(21) Anmeldenummer: **88730013.5**

(22) Anmeldetag: **18.01.88**

(51) Int. Cl.$^4$: **C 08 G 73/04**
C 08 G 69/10, C 07 K 13/00,
A 61 K 49/00, A 61 K 31/80,
A 61 K 39/385

(30) Priorität: **19.01.87 DE 3701665**

(43) Veröffentlichungstag der Anmeldung:
**03.08.88 Patentblatt 88/31**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Gries, Heinz, Dr.**
**Helmstedter Strasse 19**
**D-1000 Berlin 31 (DE)**

**Deutsch, Julius, Dr.**
**Horstweg 25**
**D-1000 Berlin 19 (DE)**

**Klieger, Erich, Dr.**
**Augsburgerstrasse 25**
**D-1000 Berlin 30 (DE)**

**Niedballa, Ulrich, Dr.**
**Gosslerstrasse 28a**
**D-1000 Berlin 33 (DE)**

**Renneke, Franz-Josef, Dr.**
**Hochstrasse 67**
**D-4709 Bergkamen (DE)**

**Conrad, Jürgen, Dr.**
**Sonnenallee 70**
**D-1000 Berlin 44 (DE)**

**Mützel, Wolfgang, Dr.**
**Weddigenweg 74**
**D-1000 Berlin 45 (DE)**

(54) **Polymer-Komplexe, Verfahren zu deren Herstellung und diese enthaltende pharmazeutische Mittel.**

(57) Polymer-Komplexe bestehend aus einem Carbonsäure-, Phosphonsäure- und/oder Phenolgruppen enthaltenden Liganden mit alkylierten Amineinheiten, mindestens einem Ion eines Elements der Ordnungszahlen 21 - 29, 31, 32, 38, 39, 42 - 44, 49 oder 57 - 83 sowie gegebenenfalls Kationen anorganischer und/oder organischer Basen, Aminosäuren, oder Aminosäureamide, sind wertvolle diagnostische und therapeutische Mittel.

EP 0 277 088 A2

**Beschreibung**

**Polymer-Komplexe, Verfahren zu deren Herstellung und diese enthaltende pharmazeutische Mittel**

Die Erfindung betrifft den in den Patentansprüchen gekennzeichneten Gegenstand, das heißt neue Polymer-Komplexe, diese Verbindungen enthaltende Mittel, ihre Verwendung in Diagnostik und Therapie sowie Verfahren zur Herstellung dieser Verbindungen und Mittel.

Die Anwendung von Komplexbildnern oder Komplexen bzw. deren Salzen in der Medizin ist seit langem bekannt. Als Beispiele seien genannt:

Komplexbildner als Stabilisatoren pharmazeutischer Präparate, Komplexe und deren Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen, als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotope wie $^{99m}Tc$ für die Szintigraphie sind bekannt.

In der Patentschrift DE-OS 3401052 sind neuerdings paramagnetische Komplexsalze als Diagnostika, vorwiegend als NMR-Diagnostika vorgeschlagen worden.

Diese Komplexe oder Komplexsalze sind recht gut verträglich und gewährleisten eine weitestgehend vollständige Ausscheidung der paramagnetischen Ionen. Der Nachteil ist allerdings, daß sie sich nur unspezifisch im Extrazellulärraum verteilen und daher nur im Ausnahmefall für eine Erkennung pathologisch veränderter Gewebe taugen.

Der Ansatz, zumindest einen Teil dieser Probleme durch Verwendung von Komplexbildnern, die einerseits durch ionische Bindung an das jeweils geeignete Metall (siehe unten) sowie andererseits durch Bindung an eine funktionelle Gruppe oder ein als Carrier-Molekül dienendes nicht-toxisches und möglichst organ-spezifisches Molekül gebunden sind, zu lösen, war bisher nur sehr begrenzt erfolgreich.

So ist beispielsweise die Anzahl paramagnetischer Zentren in den Komplexen, die in den Europäischen Patentanmeldungen No. 88 695 und No. 150 884 beschrieben sind, für eine organspezifische Bildgebung nicht ausreichend.

Erhöht man die Anzahl der benötigten Metallionen durch mehrfache Einführung komplexierender Einheiten in ein Makromolekül, so ist das immer mit einer nicht tolerierbaren Beeinträchtigung der Affinität und/oder Spezifizität dieses Makromoleküls verbunden [J. Nucl. Med. 24 1158 (1983)].

Es besteht daher für vielfältige Zwecke ein Bedarf an stabilen, gut löslichen, aber auch besser verträglichen, gut zugänglichen Komplexverbindungen, die eine möglichst große Anzahl der benötigten Metallionen im Komplex enthalten, ohne daß ihre Affinität und/oder Spezifizität verloren geht. Der Erfindung liegt somit die Aufgabe zugrunde, diese Verbindungen und Mittel zur Verfügung zu stellen, sowie ein möglichst einfaches Verfahren zu ihrer Herstellung zu schaffen. Diese Aufgabe wird durch die Erfindung gelöst.

Es wurde gefunden, daß sich Polymer-Komplexe, die aus einem Carbonsäure-, Phosphonsäure- und/oder Phenolgruppen enthaltenden Liganden mit alkylierten Amineinheiten, mindestens einem Ion eines Elements der Ordnungszahlen 21 - 29, 31, 32, 38, 39, 42 - 44, 49 oder 57 - 83 sowie gegebenenfalls Kationen anorganischer und/oder organischer Basen, Aminosäuren oder Aminosäureamide bestehen, überraschenderweise hervorragend zur Herstellung von NMR-, Röntgen- und Radio -Diagnostika sowie Radiotherapeutika eignen, da sie vor allem die für diese Verwendung benötigte Anzahl von Metallionen im Komplex stabil gebunden enthalten.

Als Ligand werden zum Beispiel Verbindungen der allgemeinen Formel I

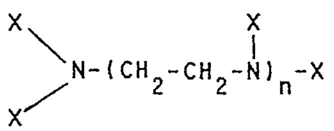

(I)

worin

n die ganzen Ziffern 7 bis 20.000,

X ein Wasserstoffatom, ein $CR^1R^2R^3$- oder ein $CR^1R^2$- U-Rest mit U in der Bedeutung eines

U in der Bedeutung eines 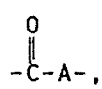, 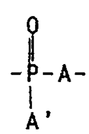 oder 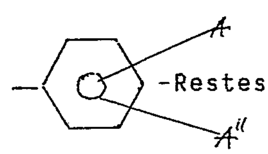-Restes,

worin

A′ für OH oder A und A″ für H oder A′ stehen, mit

A in der Bedeutung einer $(NH)_u$-$[B$-$(NH)_v]_w$-W- oder $OR^4$-Gruppe, worin

u und v für die Ziffern 0, 1 oder 2,

w für die Ziffern 0 oder 1,
$R^1$ für eine $(CH_2)_m$ Z-Gruppe,
$R^2$ für ein Wasserstoffatom oder eine $(CH_2)_l$ Z-Gruppe,
$R^3$ für ein Wasserstoffatom oder eine $(CH_2)_k$ Z-Gruppe und
$R^4$ für einen $C_1$-$C_6$-Alkyl- oder Benzylrest stehen,
wobei
m, l und k für die Ziffern 0 bis 10 und
Z für ein Wasserstoffatom, eine $C_1$-$C_{20}$-Alkyl-, eine COOH-, $PO_3H_2$- oder

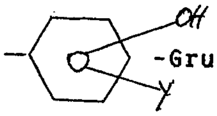 -Gruppe

stehen, worin Y ein Wasserstoffatom oder den Rest OH bedeutet,
B eine $C_0$-$C_{20}$-Alkylengruppe und
W ein Wasserstoffatom, ein Makromolekül, eine gegebenenfalls über eine geradkettige, verzweigte, gesättigte oder ungesättigte $C_1$-$C_{20}$-Alkylengruppe gebundene funktionelle Gruppe oder gebunden über diese funktionelle Gruppe ein Makro- oder Biomolekül, wobei die Alkylengruppe gegebenenfalls Imino-, Phenylenoxy-, Phenylenimino-, Amid-, Estergruppe(n), Sauerstoff-, Schwefel- und/oder Stickstoff-Atom(e) enthalten und gegebenenfalls durch Hydroxy-, Mercapto- und/oder Aminogruppe(n) substituiert ist, bedeuten,
wobei die Substituenten X in den einzelnen Gliedern und an den Enden der Verbindung sowie die Substituenten Z in $R^1$, $R^2$ und $R^3$ gleich oder verschieden sein können, mit den Maßgaben, daß nicht alle X gleichzeitig Wasserstoff bedeuten sollen, daß zumindest ein Z nicht Wasserstoff oder $C_1$-$C_{20}$-Alkyl bedeutet, daß im Falle von m, l und k = 0 nicht alle Substituenten Z für -COOH, $-PO_3H_2$ oder

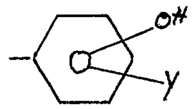

stehen und daß gewünschtenfalls ein Teil der -COOH-, $-PO_3H_2$- oder

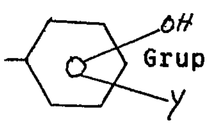 Gruppen

als Ester oder Amide vorliegt,
und II,

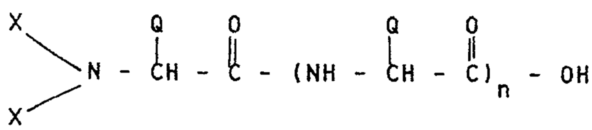 (II),

worin
Q den Rest einer, die Gruppe(n) NX und/oder $NX_2$ enthaltenden natürlichen Aminosäure bedeutet und
n und X die oben genannten Bedeutungen haben,
eingesetzt.

Das Element der oben genannten Ordnungszahl, welches das Zentralion des physiologisch verträglichen Komplexsalzes bildet, kann für den angestrebten Verwendungszweck des erfindungsgemäßen diagnostischen Mittels selbstverständlich auch radioaktiv sein.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21-29, 42, 44 und 57-70. Geeignete Ionen sind beispielsweise das Chrom(III)-, Mangan (II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium (III)-, Dysprosium(III)-, Holmium(III)-, Erbium(III)- und Eisen(III)-ion.

Für die Verwendung der erfindungsgemäßen Mittel in der Nuklearmedizin muß das Zentralion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium und Iridium.

Ist das erfindungsgemäße Mittel zur Anwendung in der Röntgen-Diagnostik bestimmt, so muß sich das

Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Die erfindungsgemäßen Polymer-Komplexe enthalten mindestens ein Ion eines Elements der oben genannten Ordnungszahl. Bevorzugt sind Komplexe, die ein Ion auf 5-50, insbesondere 8-20 COOH-, $PO_3H_2$-oder Phenolgruppen aufweisen.

Die in W enthaltene sowie die für B stehende Alkylengruppe kann geradkettig, verzweigt, cyclisch, aliphatisch, aromatisch oder aralaliphatisch sein und bis zu 20 Kohlenstoffatome aufweisen. Bevorzugt sind geradkettige Mono-, bis Hexamethylengruppen sowie $C_1$-$C_4$-Alkylenphenylgruppen.

Die in Z bzw. $R^4$ enthaltene Alkylgruppe kann geradkettig, verzweigt oder cyclisch sein und bis zu 20 bzw. 6 Kohlenstoffatome aufweisen. Bevorzugt sind geradkettige $C_1$-$C_6$- bzw. $C_1$-$C_4$-Reste. Enthält der erfindungsgemäße Polymer-Komplex den Rest $CR^1R^2$-U, so sind pro Molekül 1 bis n/10, bevorzugt 1 bis n/50, dieser Reste vorhanden.

Bevorzugte, für den Substituenten W stehende funktionelle Gruppen sind beispielsweise die Maleimidobenzoyl-, 3-Sulfomaleimidobenzoyl-, 4-(Maleimidomethyl)cyclohexylcarbonyl-, 4-[3-Sulfo-(maleimidomethyl)-cyclohexyl-carbonyl-, 4-(p-Maleimidophenyl)-butyryl-, 3-(2-Pyridyldithio)propionyl-, Methacryloyl-(pentamethylen)amino-, Bromacetyl-, Jodacetyl-, 3-Jodpropyl-, 2-Bromethyl-, 3-Mercaptopropyl-, 2-Mercaptoethyl-, Phenylenisothiocyanat-, 3-Aminopropyl-, Benzylester-, Ethylester-, t-Butylester-, Amino-, $C_1$-$C_6$-Alkylamino-, Aminocarbonyl-, Hydrazino-, Hydrazinocarbonyl-, Maleimido-, Methacrylamido-, Methacryloylhydrazinocarbonyl-, Maleimidamidocarbonyl-, Halogeno-, Mercapto-, Hydrazinotrimethylenhydrazinocarbonyl-, Aminodimethylenamidocarbonyl-, Bromcarbonyl-, Phenylendiazonium-, Isothiocyanat-, Semicarbazid-, Thiosemicarbazid-Gruppe.

Zur Verdeutlichung seien einige ausgewählte Gruppen aufgeführt:

$-CH_2-C_6H_4-O(CH_2)_3-$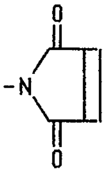 , $-CH_2-C_6H_4-O(CH_2)_3NH$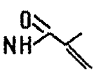, $-NH-$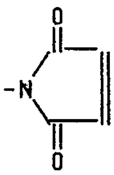,

$-CH_2-C_6H_4-O(CH_2)_5CO_2CH_2C_6H_5$, $-CH_2-C_6H_4-O-CH_2-CO_2CH_2C_6H_5$,

$-CH_2-C_6H_4-O(CH_2)_5CONHNH_2$, $-CH_2-C_6H_4-CONHNH$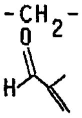, $-CH_2-C_6H_4-O(CH_2)_4-SH$,

$-CH_2-C_6H_4-O(CH_2)_3NHNH_2$, $-CH_2-C_6H_4-O(CH_2)_5-CONH-$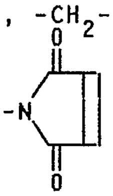 , $-CH_2-C_6H_4-O(CH_2)_3Br$,

$-CH_2-C_6H_4-O(CH_2)_5CONHNH-(CH_2)_3-NHNH_2$, $-CH_2-NHNH_2$, $-CH_2-SH$, $-CH_2CONHNH_2$,
$-(CH_2)_3SH$, $-CH_2-C_6H_4-O-CH_2COBr$, $-C_6H_4NHCOCH_2Br$,

$-CH_2-C_6H_4-OCH_2-\overset{O}{\overset{\|}{C}}-NH-(CH_2)_2NH_2$, $-CH_2-C_6H_4-NH_2$, $-C_6H_4-N_2$, $-C_6H_4NHCS$,

$-CH_2-C_6H_4-NH-\overset{O}{\overset{\|}{C}}-(CH_2)_2-S-S-$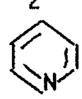 , $-NHCO-NH-NH_2$, $-NHCS-NH-NH_2$,

$-CH_2-C_6H_4-O-CH_2-$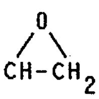, $-CH_2-C_6H_4-O-CH_2-\overset{O}{\overset{\|}{C}}-NH-(CH_2)_{10}-\overset{O}{\overset{\|}{C}}-NHNH_2$,

$-CH_2-C_6H_4-O-CH_2-CHOH-CH_2-NH(CH_2)_{10}-\overset{O}{\overset{\|}{C}}-NHNH_2$, $-OCH_2-\overset{O}{\overset{\|}{C}}-\overset{CH_3}{\overset{|}{N}}-CH_2-(CHOH)_4-CH_2OH$,

$-CH_2-$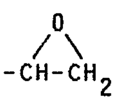, $-CH_2-O-(CH_2)_3-$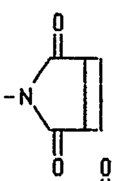, $-CH_2-O-(CH_2)_4-SH$,

$-CH_2-O-(CH_2)_3-NHNH_2$, $-CH_2-O-CH_2-\overset{O}{\overset{\|}{C}}-NH-NH_2$, $-CH_2-O-CH_2-CH_2-NH_2$,

$-CH_2-O-CH_2-NH-\overset{O}{\overset{\|}{C}}-(CH_2)_2-S-$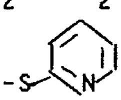 , $CH_2-O-CH_2-\overset{O}{\overset{\|}{C}}-NH-(CH_2)_{10}-\overset{O}{\overset{\|}{C}}-NH-NH_2$,

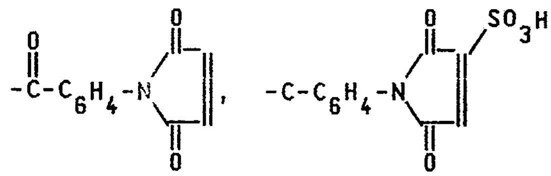, $-C\equiv C-C\equiv C-R$, $-C\equiv C-CH=CRR'$,

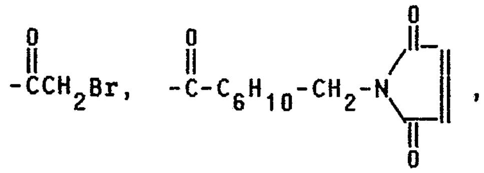,

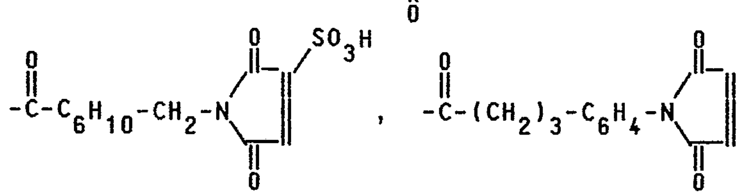, 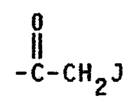, $-(CH_2)_3SH$,

$-(CH_2)_3NH_2$, $-C_6H_4SCN$, 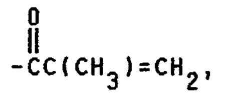, 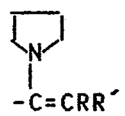, $C_6H_4CH_2Br$,

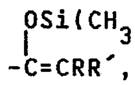, $-CH_2Br$, $-CH_2J$, $-CH=CH-CH_2Br$, $-OSO_2C_6H_4CH_3$, $-SO_2Cl$, $-SOCl$,

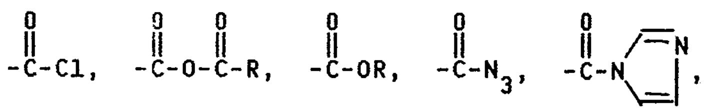, $-CH=CH-CO_2R$,

wobei R und R′ gleich oder verschieden sind und jeweils für ein Wasserstoffatom, einen gesättigten oder ungesättigten gegebenenfalls durch eine Phenylgruppe substituierten $C_1$-$C_{20}$-Alkylrest oder eine Phenylgruppe stehen.

Als Beispiele für die in Formel II enthaltenen Aminosäuren seien Lysin, Arginin, Ornithin, α,γ-Diaminobuttersäure und Histidin genannt.

Die restlichen aziden Wasserstoffatome, das heißt diejenigen, die nicht durch das Zentralion substituiert worden sind, können gegebenenfalls durch Ester- oder Amidreste oder durch Kationen anorganischer und/oder organischer Basen oder Aminosäuren ersetzt sein. Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin,
Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.
Geeignete Alkylreste sind vor allem die $C_1$-$C_6$-Alkylreste wie zum Beispiel Methyl, Ethyl, Propyl, Butyl sowie auch der Benzylrest.
Geeignete Amidreste sind vor allem die durch Umsetzung mit $C_1$-$C_6$-Mono- und Dialkylaminen wie zum Beispiel Dimethyl-, Diethyl- Dipropyl-, Cyclopropyl-, N-Methyl-n-propyl, N-Ethylcyclohexylamin, durch Umsetzung mit zum Beispiel Benzylamin, Anilin oder heterocyclischen Aminen, wie zum Beispiel Morpholin, Piperidin, oder durch Umsetzung mit hydroxylierten Aminen, wie zum Beispiel 2,3-Dihydroxypropylamin, N-Methyl-2,3-dihydroxypropylamin, 2-Hydroxy-1-(hydroxymethyl)-ethylamin, N,N-Bis-(2-hydroxyethyl)-amin, N-Methyl-2,3,4,5,6-pentahydroxyhexylamin, 6-Amino-2,2-dimethyl-1,3-dioxepin-5-ol, 2-Hydroxyethylamin, 2-Amino-1,3-propandiol, Diethanolamin, Ethanolamin gebildeten Reste.

Die erfindungsgemäßen Polymer-Komplexe enthalten die für ihre Verwendung benötigte große Anzahl von Metallionen im Komplex stabil gebunden.

So ergeben z.B. Gleichgewichts- bzw. Umkomplexierungs-Untersuchungen mit dem als Stand der Technik anzusehendem, in der Fachwelt als gutes Kontrastmittel anerkanntem Gadolinium-Komplex der Diethylentriaminpentaessigsäure DTPA (Europäische Patentschrift EP 71 564), daß die erfindungsgemäßen Polymer-Komplexe überraschenderweise über den gesamten durch die Indexziffern bestimmten Molekularbereich stabiler sind als dieser.

Auch die Verträglichkeit der Polymer-Komplexe ist beispielsweise der organspezifischer monomerer Komplexe überlegen.

Überraschend hoch ist der Wert für die ein Maß der Bildgebung darstellende Größe der Relaxivität: Sie ist für die erfindungsgemäßen Polymer-Komplexe um den Faktor 10 höher als für Gadolinium-DTPA, bezogen auf die Menge an komplexiertem Metall. Nimmt man als Vergleich einen Polymer-Komplex, dessen Größe durch einen Wert von z.B. 5000 für n bestimmt sein soll, so resultiert pro Molekül Polymer-Komplex eine ca. 5000 - 10.000fach größere Relaxivität als bei Gadolinium-DPTA. Hierdurch wird, um eine bestimmte Signalstärker bei der Bildgebung zu erhalten, eine entsprechend geringere molare Menge an Komplex und damit an absoluter Menge Gadolinium im Vergleich zu den Monomeren benötigt.

Als weiterer wichtiger Vorteil der erfindungsgemäßen Polymer-Komplexe ist ihr Ausscheidungsverhalten anzuführen. So wird beispielsweise bei einem Komplex mit einem mittleren Molekulargewicht von 60.000 D und einem Gewichtsanteil an Gadolinium von 15 % innerhalb von 4 Stunden 80 % der gesamten Menge über den Harn ausgeschieden. Die je nach Anwendungszweck erwünschte Ausscheidungsgeschwindigkeit läßt sich dabei sehr gezielt und einfach über das zu wählende Molekulargewicht einstellen.

Die erfindungsgemäßen Polymer-Komplexe weisen eine überraschend hohe Gewebespezifität auf. So erhält man beispielsweise bereits wenige Minuten nach intravenöser Injektion einer N-Methylglucaminsalzlösung vom Gadolinium(III)-Komplex der Polyethylenimin-polyessigsäure im Kernresonanzbild eine deutliche Kontrastverstärkung in peripherem Tumorgewebe, die längere Zeit anhält und einen deutlichen diagnostischen Zugewinn bringt.

Überraschenderweise können auch mit Hilfe der erfindungsgemäßen Polymer-Komplexe Blutgefäße ohne Anwendung spezieller Puls-Sequenzen in-vivo dargestellt werden.

Die Herstellung der erfindungsgemäßen Polymer-Komplexe erfolgt ausgehend von Carbonsäure-, Phosphonsäure- und/oder Phenolgruppen enthaltenden Edukten, die in an sich bekannter Weise durch Alkylierung (J. March, Advanced Organic Chemistry, McGraw-Hill, 2nd ed. 1977 377 - 382) von aminogruppentragenden Liganden, z.B. mit Bromessigsäure, erhalten werden.

Die folgenden Reaktionsstufen werden ebenfalls nach literaturbekannten Methoden durchgeführt. Es sind dies die Komplexierung mit mindestens einem Ion eines Elements der oben genannten Ordnungszahlen und, falls Verbindungen mit $CR^1R^2U$-Rest(en) synthetisiert werden sollen, Überführung mindestens eines Teils der nicht zur Komplexierung benötigten Carbonsäure-, Phosphonsäure- oder Phenolgruppen in die Gruppe(n) $CR^1R^2U'$, worin U' die für U angegebene Bedeutung hat, nur daß anstelle von W der Rest W' mit den Definitionen Wasserstoffatom oder eine gegebenenfalls über eine $C_1$-$C_{20}$-Alkylengruppe gebundene funktionelle Gruppe steht - wobei die so erhaltenen $C'R^1R^2U'$-Gruppen gleich oder verschieden (z.B. Ester- oder Amid- und Hydrazidgruppen) sein können - gewünschtenfalls Bindung eines Makromoleküls sowie gegebenenfalls anschließend Substitution der restlichen noch vorhandenen aziden Wasserstoffatome durch Kationen anorganischer und/oder organischer Basen oder Aminosäuren.

Bei der Synthese der die Gruppe(n) $-CR^1R^2-U'$ bzw. $-CR^1R^2U$ enthaltenen Polymer-Komplexe können die angegebenen Stufen Komplexierung, Funktionalisierung, Bindung an ein Makromolekül permutiert werden, wobei natürlich einer Bindung an ein Makromolekül die Funktionalisierung der Carbonsäure-, Phosponsäure- oder Phenolgruppe(n) vorausgehen muß.

Die Einführung des Restes A", in die Carbonsäure-, Phosphonsäure- oder Phenol-Gruppe(n), d.h. von Amino-, Hydrazino- und Estergruppen sowie der in W und W' enthaltenen gegebenenfalls über eine $C_1$-$C_{20}$-Alkylengruppe gebundenen funktionellen Gruppe erfolgt nach literaturbekannten Verfahren (Houben-Weyl, Methoden der organischen Chemie, Band VIII, Georg Thieme Verlag, Stuttgart; J. Biochem. 92, 1413 (1982).

Beispiele für die Umwandlung von an aromatische oder aliphatische Reste gebundenen Aminogruppen sind die in wasserfreien, aprotischen Lösungsmitteln wie Tetrahydrofuran, Dimethoxyethan oder Dimethylsulfoxid in Gegenwart eines Säurefängers wie z.B. Natriumhydroxid, Natriumhydrid oder Alkali- oder Erdalkalicarbonaten wie z.B. Natrium-, Magnesium-, Kalium-, Calciumcarbonat bei Temperaturen zwischen 0 °C und dem Siedepunkt des jeweiligen Lösungsmittels, vorzugsweise jedoch zwischen 20 °C und 60 °C, durchgeführten Umsetzungen mit einem Substrat der allgemeinen Formel III

Nf-L-Fu (III),

worin Nf für ein Nucleofug wie z.B. Cl, Br, J oder $CH_3C_6H_4SO_3$, L für einen aliphatischen, aromatischen, arylaliphatischen, verzweigten, geradkettigen oder cyclischen Kohlenwasserstoffrest mit bis zu 20 Kohlenstoffatomen und Fu für die gewünschte endständige funktionelle Gruppe, gegebenenfalls in geschützter Form stehen (DE-OS 34 17 413).

Als Beispiele für Verbindungen der allgemeinen Formel III seien genannt

$BrCH_2COOCH_3$, $BrCH_2CO_2{}^tBu$,

$Br(CH_2)_2NH_2$, $Br(CH_2)_3OH$, $BrCH_2COOCH_3$, $BrCH_2CO_2{}^tBu$, $BrCH_2$- 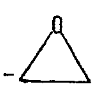

$Br(CH_2)_4CO_2C_2H_5$, $BrCH_2COBr$, $BrCH_2CONH_2$, $ClCH_2COOC_2H_5$,

$ClCH_2CONHNH_2$.

Umwandlungen von Carboxy-Gruppen können zum Beispiel nach der Carbodiimid-Methode (Fieser,

Reagents for Organic Syntheses 10,142) über ein gemischtes Anhydrid [Org. Prep. Proc. Int. 7,215 (1975)] oder über einen aktivierten Ester (Adv. Org. Chem., Part B, 472) durchgeführt werden.

Die so erhaltenen komplexbildenden Liganden (sowie die Komplexe) können auch an Bio- oder Makromoleküle geknüpft sein, von denen bekannt ist, daß sie sich in dem zu untersuchenden Organ oder Organteil besonders anreichern. Solche Moleküle sind beispielsweise Enzyme, Hormone, Dextrane, Porphyrine, Bleomycine, Insulin, Prostaglandine, Steroidhormone, Aminozucker, Aminosäuren, Peptide wie Polylysin, Proteine (wie zum Beispiel Immunoglobuline, monoklonale Antikörper, Lektine oder Lipide (auch in Form von Liposomen). Besonders hervorzuheben sind Konjugate mit Albuminen, wie Humanserumalbumin, Antikörpern, wie zum Beispiel monoklonale, für tumorassoziierte Antigene spezifische Antikörper oder Antimyosin. Anstelle von biologischen Makromolekülen können auch geeignete synthetische Polymere wie Polyethylenimine , Polyamide, Polyharnstoffe und Polythioharnstoffe angeknüpft werden. Die hieraus gebildeten pharmazeutischen Mittel eignen sich beispielsweise zur Anwendung in der Tumor- und Infarkt-Diagnostik sowie Tumortherapie. Als monoklonale Antikörper (zum Beispiel Nature 256, 495, 1975), die gegenüber den polyklonalen Antikörpern die Vorzüge haben, daß sie spezifisch für eine antigene Determinante sind, eine definierte Bindungsaffinität besitzen, homogen sind (damit wird ihre Reindarstellung wesentlich einfacher) und in Zellkulturen in großen Mengen herstellbar sind, kommen für die Konjugation insbesondere solche infrage, die gegen überwiegend zellmembranständige Antigene gerichtet sind. Als solche sind zum Beispiel für die Tumordarstellung monoklonale Antikörper bzw. deren Fragmente Fab und $F(ab')_2$ geeig net, die zum Beispiel spezifisch sind für humane Tumore des Gastrointestinaltraktes, der Brust, der Leber, der Blase, der Keimdrüsen und von Melanomen (Cancer Treatment Repts. 68, 317, 1984, Bio Sci 34, 150, 1984) oder gegen Carcinoembryonales Antigen (CEA), Humanes Choriogonadotropin (β-HCG) oder andere tumorständige Antigene, wie Glycoproteine, gerichtet sind (New Engl. J. Med. 298, 1394, 1973, US-P 4,331,647). Geeignet sind unter anderem auch Anti-Myosin, Anti-Insulin- und Anti-Fibrin-Antikörper (US-P 4,036,945).

Coloncarcinome lassen sich mit Hilfe von mit Gadolinium(III)-Ionen komplexierten Konjugaten von Polyethylenimin-polyessigsäuren mit dem Antikörper 7 B5 D11 NMR-diagnostisch nachweisen.

Für Leberuntersuchungen beziehungsweise für die Tumordiagnostik eignen sich beispielsweise Konjugate oderk Einschlußverbindungen mit Liposomen, die beispielsweise als unilamellare oder multilamellare Phosphatidylcholin-Cholesterol-Vesikel eingesetzt werden.

Die Bindung von Metallen an die gewünschten Makro- oder Biomoleküle erfolgte bisher nach Methoden, wie sie zum Beispiel in Rev. Roum. Morphol. Embryol. Physio., Physiologie 1981, 18, 241 und J. Pharm. Sci. 68, 79 (1979) beschrieben sind, beispielsweise durch Reaktion der nucleophilen Gruppe eines Makromoleküls, wie der Amino-, Phenol-, Sulfhydryl-, Aldehyd- oder Imidazol-Gruppe mit einem aktivierten Derivat des Polymer-Komplexes oder Liganden. Als aktivierte Derivate kommen beispielsweise Anhydride, Säurechloride, gemischte Anhydride (siehe zum Beispiel G.E. Krejcarek und K.L. Tucker, Biochem. Biophys. Res. Commun. 1977, 581) aktivierte Ester, Nitrene oder Isothiocyanate in Betracht. Umgekehrt ist es auch möglich, ein aktiviertes Makromolekül mit dem Polymer-Komplex oder Liganden umzusetzen. Zur Konjugation mit Proteinen bieten sich auch Substituenten zum Beispiel der Struktur $C_6H_4N_2^+$, $C_6H_4NHCOCH_2$, $C_6H_4NHCS$ oder $C_6H_4OCH_2CO$ an.

Diese Art der Bindung ist jedoch mit dem Nachteil mangelnder Komplex-Stabilität der Konjugate bzw. mangelnder Spezifität behaftet (zum Beispiel Diagnostic Imaging 84, 56; Science 220, 613, 1983; Cancer Drug Delivery 1, 125, 1984).

Die Konjugatbildung gemäß vorliegender Erfindung erfolgt dagegen über die im Substituenten A befindliche Amino- bzw. Hydrazinofunktion oder über die weiter oben definierte funktionelle Gruppe im Substituenten W. Es können hierbei über eine Bindungsstelle im Makromolekül bis zu mehrere Hundert Metallionen gebunden werden.

Im Falle der Antikörper-Konjugate darf die Bindung des Antikörpers an den Komplex oder Liganden nicht zum Verlust oder zur Verminderung der Bindungsaffinität und Bindungsspezifität des Antikörpers zum Antigen führen. Dies kann entweder durch Bindung an den Kohlenhydrat-Anteil im Fc-Teil des Glycoproteins bzw. in den Fab oder $F(ab')_2$-Fragmenten oder durch Bindung an Schwefelatome des Antikörpers bzw. der Antikörper-Fragmente erfolgen.

Im ersten Fall muß zunächst eine oxidative Spaltung von Zuckereinheiten zur Generation kopplungsfähiger Formylgruppen durchgeführt werden. Diese Oxidation kann auf chemischem Wege mit Oxidationsmitteln wie z.B. Perjodsäure, Natriummetaperjodat oder Kaliummetaperjodat nach literaturbekannten Methoden (zum Beispiel J. Histochem and Cytochem. 22, 1084, 1974) in wäßriger Lösung in Konzentrationen von 1 bis 100, vorzugsweise 1 bis 20 mg/ml, und einer Konzentration des Oxidationsmittels zwischen 0,001 bis 10 mMol, vorzugsweise 1 bis 10 mMol in einem pH-Bereich von ca. 4 bis 8 bei einer Temperatur zwischen 0 bis 37 °C und einer Reaktionsdauer zwischen 15 Minuten und 24 Stunden vorgenommen werden. Auch auf enzymatischem Wege kann die Oxidation, beispielsweise mit Hilfe von Galaktoseoxidase in einer Enzymkonzentration von 10-100 Einheiten/ml, einer Substratkonzentration von 1 bis 20 mg/ml, bei einem pH-Wert von 5 bis 8, einer Reaktionsdauer von 1 bis 8 Stunden und einer Temperatur zwischen 20 und 40 °C, durchgeführt werden (zum Beispiel J. Biol. Chem. 234, 445, 1959).

An die durch Oxidation generierten Aldehyde werden Komplexe oder Liganden mit geeigneten funktionellen Gruppen wie zum Beispiel Hydrazin, Hydrazid, Hydroxylamin, Phenylhydrazin, Semicarbazid und Thiosemicarbazid durch Reaktion zwischen 0 - 37 °C, bei einer Reaktionsdauer von 1 bis 65 Stunden, einem pH-Wert

zwischen ca. 5,5 und 8, einer Antikörperkonzentration von 0,5 bis 20 mg/ml und einem molaren Verhältnis des Komplexbildners zum Antikörperaldehyden von 1:1 bis 1000:1 gebunden. Die anschließende Stabilisierung des Konju gates efolgt durch Reduktion der Doppelbindung, z.B. mit Natriumborhydrid oder Natriumcyanoborhydrid; das Reduktionsmittel wird dabei in einem 10 bis 100-fachen Überschuß verwendet (zum Beispiel J. Biol. Chem. 254, 4359, 1979).

Die zweite Möglichkeit der Bildung von Antikörper-Konjugaten geht aus von einer schonenden Reduktion der Disulfid-Brücken des Immunoglobulin-Moleküls; hierbei werden die empfindlicheren Disulfid-Brücken der H-Ketten des Antikörper-Moleküls gespalten, während die S-S-Bindungen der Antigen-bindenden Region intakt bleiben, so daß praktisch keine Verminderung der Bindungsaffinität und -spezifität des Antikörpers eintritt (Biochem. 18, 2226, 1979, Handbook of Experimental Immunology, Vol. 1, Second Edition, Blackwell Scientific Publications, London 1973, Chapter 10). Diese freien Sulfhydryl-Gruppen der intra-H-Ketten-Regionen werden dann mit geeigneten funktionellen Gruppen von Komplexbildnern oder Metallkomplexen bei 0 bis 37 °C, einem pH-Wert von ca. 4 bis 7, und einer Reaktionsdauer von 3 bis 72 Stunden unter Ausbildung einer kovalenten Bindung, die die Antigen-Bindungsregion des Antikörpers nicht beeinflußt, umgesetzt. Als geeignete reaktive Gruppen seien beispielsweise genannt: Halogenalkyl-, Halogenacetyl-, p-Mercuribenzoatgruppen sowie Gruppen, die einer Michael-Additions-Reaktion, wie zum Beispiel Maleinimide, Methacrylogruppen (zum Beispiel J. Amer. Chem. Soc. 101, 3097, 1979), zu unterwerfen sind.

Zur Verknüpfung der Antikörpersfragmente mit den Polymer-Komplexen bzw. den Liganden gibt es zusätzlich eine Reihe geeigneter, oft auch kommerziell erhältlicher bifunktioneller "Linker" (siehe zum Beispiel Pierce, Handbook and General Catalogue 1986), die sowohl gegenüber den SH-Gruppen der Fragmente als auch gegenüber den Amino- bzw. Hydrazinogruppen der Polymer reaktiv sind.
Als Beispiele seien genannt:
m-Maleimidobenzoyl-N-hydroxysuccinimidester (MBS),
m-Maleimidobenzoyl-N-sulfosuccinimidester (Sulfo-MBS),
N-Succinimidyl-[4-(Iodacetyl)-amino]benzoesäureester (SIAB),
Succinimidyl-4(N-maleimidomethyl)-cyclohexan-1-carbonsäureester (SMCC),
Succinimidyl-4(p-maleimidophenyl)-buttersäureester (SMPB),
N-Succinimidyl-3-(2-pyridyldithio)-propionsäureester (SDPD),
4-[3-(2,5-Dioxo-3-pyrrolinyl)-propionyloxy]-3-oxo-2,5-diphenyl-2,3-dihydrothiopen-1,1-dioxid.

Es können auch Bindungen nicht-kovalenter Art zur Kopplung genutzt werden, wobei sowohl ionische als auch van der Waals- und Wasserstoffbrücken-Bindungen in wechselnden Anteilen und Stärke (Schlüssel-Schloß-Prinzip) zur Bindung beitragen können (zum Beispiel Avidin-Biotin, Antikörper-Antigen). Auch Einschlußverbindungen (host-guest) kleinerer Komplexe in größere Cavitäten beim Makromolekül sind möglich.

Das Kopplungsprinzip besteht darin, zunächst ein bifunktionelles Makromolekül herzustellen, indem man entweder ein gegen ein Tumorantigen gerichtetes Antikörper-Hybridom mit einem gegen einen erfindungsgemäßen Komplex gerichtetes zweiten Antikörper-Hybridom fusioniert oder die beiden Antikörper chemisch über einen Linker (beispielsweise in der im J. Amer. Chem. Soc. 101, 3097 (1979) angegebenen Weise) miteinander verknüpft oder den gegen das Tumorantigen gerichteten Antikörper, gegebenenfalls über einen Linker, an Avidin (bzw. Biotin) bindet [D.J. Hnatowich et al., J. Nucl. Med. 28, 1294 (1987)]. Anstelle der Antikörper können auch ihre entsprechenden F(ab)-bzw. $F(ab')_2$-Fragmente verwendet werden. Für die pharmazeutische Anwendung injiziert man zunächst das bifunktionelle Makromolekül, das sich am Zielort anreichert, und dann im zeitlichen Abstand die erfindungsgemäße Komplexverbindung [gegebenenfalls an Biotin (bzw. Avidin) gebunden], die in-vivo am Zielort angekoppelt wird und dort ihre diagnostische oder therapeutische Wirkung entfalten kann. Darüberhinaus können sich auch andere Kopplungsmethoden wie beispielsweise das in Protein Tailoring Food Med. Uses [Am. Chem. Soc. Symp.] (1985), 349, beschriebene "Reversible Radiolabeling" zur Anwendung kommen.

Mit der sogenannten Festphasen-Kopplung steht eine besonders einfache Methode zur Herstellung von Antikörper-Konjugaten bzw. Antikörperfragment-Konjugaten zur Verfügung: Der Antikörper wird an eine stationäre Phase (z.B. einen Ionenaustauscher), der sich zum Beispiel in einer Glassäule befindet, gekoppelt. Durch sukzessives Spülen der Säule mit einer zur Generierung von Aldehyd-Gruppen geeigneten Lösung, Waschen, Spülen mit einer Lösung des funktionalisierten Komplexes (bzw. Liganden), Waschen (wird der Ligand eingesetzt, erfolgt noch ein Spülen mit einer das Metallsalz enthaltenen Lösung, gefolgt von nochmaligem Spülen) und schließlich Eluieren des Konjugats werden sehr hohe Konjugat-Ausbeuten erhalten.

Dieses Verfahren erlaubt die automatische und kontinuierliche Produktion beliebiger Mengen an Konjugaten.

Auch andere Kopplungsschritte können auf diese Art und Weise durchgeführt werden.

So können zum Beispiel durch die Sequenz Papain-Reduktion/ bifunktioneller Linker/ funktionalisierter Komplex bzw. Ligand Fragment-Konjugate hergestellt werden.

Die so gebildeten Verbindungen werden anschließend vorzugsweise chromatographisch über Ionenaustauscher auf einer Fast-Protein-Liquid-Chromatography-Anlage gereinigt.

Die Herstellung der erfindungsgemäßen Metallkomplexe erfolgt in der Weise, wie sie in der Patentschrift DE-OS 34.01.052 offenbart worden ist, indem man das Metalloxid oder ein Metallsalz (beispielsweise das Nitrat, Acetat, Carbonat, Chlorid oder Sulfat) des Elements der Ordnungszahlen 21-29, 31, 32, 38, 39, 42-44,

49, 57-83 in Wasser und/oder einem niederen Alkohol (wie Methanol, Ethanol oder Isopropanol) löst oder suspendiert und mit der Lösung oder Suspension der äsquivalenten Menge des komplexbildenden Liganden umsetzt und anschließend, falls gewünscht, vorhandene azide Wasserstoffatome der Säure- bzw. Phenolgruppen durch Kationen anorganischer und/oder organischer Basen oder Aminosäuren substituiert.

Die Neutralisation erfolgt dabei mit Hilfe anorganischer Basen (zum Beispiel Hydroxiden, Carbonaten oder Bicarbonaten) von zum Beispiel Natrium, Kalium oder Lithium und/oder organischer Basen wie unter anderem primärer, sekundärer und tertiärer Amine, wie zum Beispiel Ethanolamin, Morpholin, Glucamin, N-Methyl-und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrenssschritt einzusparen.

Enthalten die sauren Komplexverbindungen mehrere freie azide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Konjugate aus Antikörper und Komplex werden vor der in vivo Anwendung nach Inkubation mit einem schwachen Komplexbildner, wie zum Beispiel Natriumcitrat, Natrium-Ethylen-diamintetraessigsäure dialysiert, um schwachgebundene Metallatome zu entfernen.

Im Falle der Verwendung von Radioisotope enthaltenden Komplexverbindungen kann deren Herstellung nach den in "Radiotracers for Medical Applications", Volume 1 CRC-Press, Boca Raton, Florida beschriebenen Methoden vorgenommen werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren der Galenik üblichen Hilfsstoff(en) (zum Beispiel Methylcellulose, Lactose, Mannit) und/oder Tensid(en) (zum Beispiel Lecithine, Tween®, Myrj® und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1 µMol -1 Mol/l des Metalls in Form seines Komplexsalzes und werden in der Regel in Mengen von 0,001 - 5 mMol Metall/kg dosiert. Sie sind zur enteralen und parenteralen Applikation bestimmt.

Die erfindungsgemäßen Komplexverbindungen kommen zur Anwendung

für die NMR-, Röntgen- und Ultraschall-Diagnostik in Form ihrer Komplexe mit den Ionen der Elemente mit den Ordnungszahlen 21-29, 42, 44 und 57-83;

2. für die Radiodiagnostik und Radiotherapie in Form ihrer Komplexe mit den Radioisotopen der Elemente mit den Ordnungszahlen 27, 29, 31, 32, 38, 39, 43, 49, 62, 64, 70 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist,

um den nichtinvasiven Charakter der Untersuchungen aufrechtzuerhalten.

Die gute Wasserlöslichkeit der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika Müssen 100 - 1000fach besser wasserlöslich sein als für die NMR-Spektroskopie. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen -Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,001- 5 mMol Metall/kg, vorzugsweise 0,005 -0,5mMol Metall/kg, dosiert. Details der Anwendung werden zum Beispiel in H.J. Weinmann et al., Am. J. of Roentgenology 142, 619 (1984) diskutiert.

Besonders niedrige Dosierungen (unter 1 mg/kg von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Shift-Reagenzien verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details ihrer Anwendung und Dosierung werden z.B. in "Radiotracers for Medical Applications", CRC-Press, Boca Raton, Florida beschrieben.

Eine weitere bildgebende Methode mit Radioisotopen ist die Positronen-Emissions-Tomographie, die positronenemittierende Isotope wie z.B. $^{43}Sc$, $^{44}Sc$, $^{52}Fe$, $^{55}Co$, $^{68}Ga$ und $^{81}Rb$ verwendet.

Die erfindungsgemäßen Verbindungen können auch in der Radioimmunotherapie verwendet werden. Diese unterscheidet sich von der entsprechenden Diagnostik nur durch die Menge und Art des verwendeten radioaktiven Isotops. Ziel ist dabei die Zerstörung von Tumorzellen durch energiereiche kurzwellige Strahlung mit einer möglichst geringen Reichweite. Die Spezifität des verwendeten Trägers (z.B. Chelat-Antikörper) ist dabei von entscheidender Bedeutung, da unspezifisch lokalisierte Antikörperkonjugate zur Zerstörung von gesundem Gewebe führen.

Der Antikörper bzw. das Antikörper-Fragment des erfindungsgemäßen Antikörper-Metall-Komplexes dient dazu, den Komplex immunspezifisch für das betreffende Antigen an das Zielorgan zu transportieren, wo das wegen seiner zelltötenden Eigenschaften ausgewählte Metallion Strahlen emittieren kann, die die Zellen letal schädigen. Geeignete $\beta$-emittierende Ionen sind, zum Beispiel $^{46}Sc$, $^{47}Sc$, $^{48}Sc$, $^{72}Ga$ und $^{73}Ga$. Geeignete geringe Halbwertzeiten aufweisende $\alpha$-emittierende Ionen sind zum Beispiel $^{211}Bi$, $^{212}Bi$, $^{213}Bi$ und $^{214}Bi$, wobei $^{212}Bi$ bevorzugt ist.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Portein wie zum Beispiel Human Serum Albumin verabreicht werden. Die Dosierung ist dabei abhängig von der Art der zellulären Störung und dem benutzten Metallion.

Die erfindungsgemäßen therapeutischen Mittel werden parenteral, vorzugsweise i.V., appliziert.

Details der Anwendung von Radiotherapeutika werden zum Beispiel in R.W. Kozak et al. TIBTEC, Oktober 1986, 262, diskutiert.

Die erfindungsgemäßen Mittel sind ebenfalls als Röntgenkontrastmittel geeignet, wobei besonders hervorzuheben ist, daß sie auch im Vergleich zu den bisher gebräuchlichen jodhaltigen Kontrastmitteln eine für die Diagnostik wesentlich nützlichere Pharmakokinetik erkennen lassen. Besonders wertvoll sind sie weiterhin wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 -5 mMol Metall/kg. vorzugsweise 0,25 - 1 mMol Metall/kg. dosiert.

Details der Anwendung von Röntgenkontrastmitteln werden zum Beispiel in Barke, Röntgenkontrastmittel, G. Thieme, Leipzig (1970) und P. Thurn, E. Bücheler -"Einführung in die Röntgendiagnostik", G. Thieme, Stuttgart, New York (1977) diskutiert.

Die erfindungsgemäßen Mittel sind - da ihre akustische Impedanz höher ist als die von Körperflüssigkeiten und Geweben - auch als Kontrastmittel für die Ultraschalldiagnostik geeignet, insbesondere in Form von Suspensionen. Sie werden im allgemeinen in Mengen von 0,1 bis 5 mMol/kg, vorzugsweise von 0,25 bis 1 mMol/kg dosiert.

Details der Anwendung von Ultraschalldiagnostika werden zum Beispiel in T.B. Tyler et al., Ultrasonic Imaging 3.323 (1981), J.I. Haft, "Clinical Echokardiography", Futura, Mount Kisco, New York 1978 und G. Stefan "Echokardiographie" G. Thieme Stuttgart/New York 1981, beschrieben.

Insgesamt ist es gelungen, neue Polymer-Komplexe zu synthetisieren, die neue Möglichkeiten in der diagnostischen und therapeutischen Medizin erschließen. Vor allem die Entwicklung neuartiger bildgebender Verfahren in der medizinischen Diagnostik läßt diese Entwicklung wünschenswert erscheinen.

Die nachfolgenden Beispiele dienen zur näheren Erläuterung des Erfindungsgegenstandes.

Beispiel 1

a) Gadolinium-Komplex der Polyethyleniminpolyessigsäure

37,0 g Polyethyleniminpolyessigsäure, hergestellt nach DE 2.303.081, werden in 500 ml bidest. Wasser gelöst und mit 10 g Gadoliniumcarbonat (hydratisiert, 59 Gew % Gd) unter Rühren 2 Stunden auf 80 ° erhitzt. Nach Ultrafiltration und Gefriertrocknung erhält man 41.8 g weißes, voluminöses Material, das laut Elementaranalyse 13,55 Gew % Gadolinium enthält.

Analog zur Vorschrift 1a erhält man aus Polyethyleniminpolyessigsäure und

b) Mangan-(II)-carbonat den Mangankomplex mit 5,19 Gew % Mangan
c) Cobaltcarbonat den Cobaltkomplex mit 5,45 Gew % Cobalt
d) basischem Kupfercarbonat den Kupferkomplex mit 5.74 Gew % Kupfer
e) Dysprosiumcarbonat den Dysprosium-Komplex mit 15,17 Gew % Dysprosium
f) Lanthancarbonat den Lanthankomplex mit 15,08 Gew % Lanthan
g) Yttriumcarbonat den Yttriumkomplex mit 8.14 Gew % Yttrium
h) Samariumcarbonat den Samarium-Komplex mit 16,33 Gew % Samarium
i) Holmiumcarbonat den Holmiumkomplex mit 14,12 Gew % Holmium
j) Ytterbiumcarbonat den Ytterbiumkomplex mit 17.03 Gew % Ytterbium
k) Wismutoxid den Wismutkomplex mit 17,39 Gew % Wismut
l) Eisen-(III)-oxid den Eisen-III-komplex mit 5,27 Gew % Eisen
m) Bleicarbonat den Bleikomplex mit 19,08 Gew % Blei.

Beispiel 2

a) Natriumsalz des Gadoliniumkomplexes der Polyethyleniminpolyessigsäure

12,36 g des, wie unter 1a) beschrieben hergestellten Komplexes wurden in 50 ml Wasser gelöst und mit 78,7 ml 1 N Natronlauge versetzt. Unter starkem Rühren gießt man in 2 Liter Aceton ein, filtriert den Niederschlag ab und trocknet im Vakuum bis zur Gewichtskonstanz. Man erhält 13,0 g eines weißen, körnigen Produkts, das 11,83 Gew.% Gadolinium und 12,84 Gew.% Natrium enthält.

b) Calcium-Salz des Gadolinium-Komplexes der Polyethyleniminpolyessigsäure

6,38 g des wie unter 1a) beschrieben hergestellten Komplexes werden in 100 ml Wasser gelöst und mit 2,1 g Calciumcarbonat über 5 Stunden bei 80 ° C gerührt. Nach Ultrafiltration und Gefriertrocknung erhält man 7,03 g als weißes Pulver, das 12,08 Gew.% Gadolinium und 10,81 Gew.% Calcium enthält.

c) N-Methyl-D-Glucaminsalz des Gadolinium-Komplexes der Polyethyleniminpolyessigsäure

21,35 g des wie unter 1a) beschrieben hergestellten Komplexes werden in 200 ml Wasser gelöst und mit einer Lösung, die 1 Mol N-Methyl-D-glucamin pro Liter enthält, unter stetigem Rühren bei 30 ° C solange versetzt, bis der pH-Wert bei 6,85 liegt. Nach Gefriertrocknung erhält man 47,48 g als weißes Pulver, das 6,19 Gew.% Gadolinium enthält.

d) Ethanolamin-Salz des Gadolinium-Komplexes der Polyethyleniminpolyessigsäure

3,75 g des wie unter 1a) beschrieben hergestellten Gadolinium-Komplexes werden in 50 ml Wasser gelöst und mit einer 10 %igen wässrigen Lösung von Ethanolamin versetzt, bis der pH-Wert 6,85 beträgt. Nach Gefriertrocknung erhält man 5,2 g weiße Substanz mit einem Gadoliniumgehalt von 9,84 Gew.%.

e) L-Lysin-Salz des Gadolinium-Komplexes der Polyethyleniminpolyessigsäure

2,58 g des wie unter 1a) beschrieben hergestellten Gadolinium-Komplexes werden in 50 ml Wasser gelöst und mit einer 10 %igen wässrigen L-Lysin-Lösung versetzt, bis der pH-Wert bei 6,85 liegt. Nach Gefriertrocknung bleibt das Produkt als voluminöses weißes Pulver zurück. Der Gadoliniumgehalt beträgt 7,16 Gew.%.

f) Morpholin-Salz des Gadolinium-Komplexes der Polyethyleniminpolyessigsäure

Zu einer Lösung von 3,3 g des wie unter 1a) beschrieben hergestellten Gadolinium-Komplexes fügt man so lange 10 %ige wässrige Morpholinlösung, bis der pH-Wert 6,85 beträgt. Durch Gefriertrocknung erhält man 5,1 g weißer, leicht hygroskopischer Festsubstanz mit 8,87 Gew,% Gadolinium.

Analog erhält man die entsprechenden Salze der in den Beispielen 1b) bis 1m) beschriebenen Komplexe.

Beispiel 3

a) Polyethyleniminpolyessigsäurepolyhydrazid

10,37 g Polyethyleniminpolyessigsäure werden mit 600 mg feingepulvertem Kaliumhydroxid in 150 ml trockenem Dimethylsulfoxid unter Rühren 20 Minuten auf 60 °C erwärmt. Anschließend versetzt man mit 0,5 ml Dimethylsulfat und läßt unter Rühren im Heizbad auf Raumtemperatur abkühlen. Nach 3 Stunden kühlt man auf 10 °C ab und fügt 1 ml Hydrazinmonohydrat tropfenweise zu. Danach erhitzt man noch 1 Stunde auf dem Dampfbad. Man verdünnt mit dem doppelten Volumen Wasser und dialysiert, bis im Filtrat kein Hydrazin mehr

nachweisbar ist. Nach Gefriertrocknung erhält man 8,12 g farblose Kristalle.
Hydrazin (colorimetrisch): 1,34 Mol %

b) Gadolinimumkomplex des Polyethyleniminpolyessigsäurepolyhydrazids

Man löst 3,98 g des vorstehend beschriebenen Hydrazids in 50 ml Wasser und versetzt mit halbkonzentrierter Salzsäure, bis der zwischenzeitlich entstehende Niederschlag wieder vollständig gelöst ist. Hierzu fügt man 4 ml einer 1mGadoliniumchloridlösung in 1 N Salzsäure.

Durch Zugabe festen Natriumhydroxids wird unter starken Rühren der pH-Wert auf 9 angehoben und die Lösung dialysiert. Nach Gefriertrocknung erhält man 4,51 g weiße Substanz mit einem Gadoliniumgehalt von 13,3 Gew.%.

Sie wurde über eine Säule mit Sephacryl S300 in Fraktionen unterschiedlichen Molekulargewichts getrennt. Es wurden von ihnen folgende Relaxivitäten gemessen:

| | MW | $1/T_1$ | $1/T_2$ |
|---|---|---|---|
| 1) | > 500 kD | 30,31 | 34,24 |
| 2) | 300 - 500 kD | 28,56 | 33,50 |
| 3) | 160 - 250 kD | 27,12 | 32,71 |
| 4) | 90 - 140 kD | 28,55 | 34,07 |
| 5) | 50 - 80 kD | 28,15 | 32,86 |

Alternative Darstellung des Gadoliniumkomplexes des Polyethyleniminpolyessigsäurepolyhydrazids

11,35 g des in Beispiel 2a beschriebenen Gadoliniumkomplexsalzes der Polyethylenimin-polyessigsäure werden in 250 ml wäßrigem Methanol mit 0,5 ml Dimethylsulfat versetzt und 6 Stunden gerührt. Anschließend werden 20 ml Hydrazin-monohydrat zugegeben, über Nacht gerührt und die Lösung dialysiert, bis kein freies Hydrazin mehr nachweisbar ist. Nach Gefriertrocknung erhält man 10,3 g farblose Kristalle mit einem Gadoliniumgehalt von 11,7 Gew%.
Hydrazin (colorimetrisch nach Hydrolyse): 1,28 Mol%

Beispiel 4

a) Polyethyleniminpolymalonsäure

In 250 ml Wasser 26,8 g (146,5 mMol) Brommalonsäure gelöst und vorsichtig und unter Kühlung mit 39,1 g (466 mMol) Natriumhydrogencarbonat versetzt. Dazu tropft man eine Lösung aus 4,2 g Polyethylenimin in 110 ml Wasser, wobei die Temperatur bei 40-50 °C gehalten wird. Nach 14 Stunden Rühren bei dieser Temperatur versetzt man unter Kühlen mit 30 ml conc. Salzsäure und gießt in 3 l Methanol. Der Niederschlag wird mit verdünnter Natronlauge wieder gelöst und solange gegen entionisiertes Wasser dialysiert, bis das Dialysat mit Silbernitratlösung keinen Niederschlag mehr ergibt. Nach Gefriertrocknung hinterbleibt eine gelbliche Kristallmasse.
Ausbeute: 9,63 g.
Die titrimetrische Bestimmung der Säuregruppen ergibt, daß 22 % der Stickstoffatome alkyliert sind.

b) Der Gadoliniumkomplex wird nach dem für Beispiel 1a) beschriebenen Verfahren hergestellt und enthält 7.38 Gew.% Gadolinium.

Beispiel 5

Gadoliniumkomplex der Polyethyleniminpolymalonsäurepolyessigsäure

a) 13,8 g der in Beispiel 4a) beschriebenen Polysäure werden mit 20 g Bromessigsäure und 23 g Natriumhydrogencarbonat analog zu der in 4a) beschriebenen Vorgehensweise umgesetzt. Nach Dialyse und Gefriertrocknung erhält man 17,35 g farbloser Festsubstanz.
Die Titration der Säuregrupen ergibt, daß 95 % der Stickstoffatome alkyliert sind.

b) Der Gadoliniumkomplex wird analog Beispiel 1a) erhalten. Der Gehalt an Gadolinium beträgt 13,47 Gew.%.

Beispiel 6

a) Polylysinpolyessigsäure

Eine Lösung von 13,9 g (100 mMol) Bromessigsäure in 150 ml Wasser wird unter Eiskühlung portionsweise mit 26,8 g (319 mMol) Natriumhydrogencarbonat versetzt. Dann tropft man eine Lösung von 2,5 g Polylysin (mittleres Molgewicht 30.000) in 50 ml Wasser zu, wobei man die Temperatur auf 40 - 50 °C hält. Man läßt noch über Nacht bei dieser Temperatur nachrühren, versetzt unter Eiskühlung mit 12 ml conc. Salzsäure und gießt

unter Rühren in 1,5 l Methanol ein. Der ausgeschiedene Niederschlag wird abgesaugt, feucht in verdünnter Natronlauge gelöst und so lange gegen entionisiertes Wasser dialysiert, bis das Dialysat chloridfrei ist. Durch Gefriertrocknung erhält man 4,8 g als weißes Pulver. Die Elementaranalyse zeigt, daß etwa 90 % der freien Aminogruppen alkyliert sind.

b) Gadolinium-Komplex der Polylysinpolyessigsäure

3 g der unter a) erhaltenen Verbindung werden in 50 ml Wasser mit 1,5 g Gadoliniumcarbonat, $Gd_2(CO_3)_3$, unter Rühren auf 80 °C erhitzt. Nach Membranfiltration und Gefriertrocknung der Lösung erhält man 3,8 g als weißes Pulver. Der Gehalt an Gadolinium beträgt 10,5 %, bezogen auf wasserfreie Substanz.

Beispiel 7

Gadoliniumkomplex des Konjugats des monoklonalen Antikörpers MARG11 mit Polyethyleniminpolyessigsäurepolyhydrazid

a) 4 ml einer 50 n molaren Lösung des monoklonalen Antikörpers MARG11 (Subklasse IgG/1: Cammon Laborservice GmbH, Wiesbaden) in 0,1 M-Acetatpuffer (pH 4,5) werden mit dem gleichen Volumen gepufferter 50 mM-Natriumperjodatlösung versetzt und 30 Minuten bei Raumtemperatur unter Lichtausschluß geschüttelt. Danach wird überschüssiges Perjodat mit Ethylenglykol zerstört und die Lösung dialysiert. Anschießend gibt man eine Lösung von 216 mg des in Beispiel 3 beschriebenen Polyethyleniminpolyessigsäurepolyhydrazids in 8 ml Acetatpuffer und 6 mg Natriumborhydrid zu und inkubiert 40 Stunden bei 4 °C unter Schütteln. Die Lösung wird über einen Kationenaustauscher gereinigt, gegen einen 0,1 m-Ammoniumacetatpuffer dialysiert und lyophilisiert. Man erhält 47 mg eines weißen Pulvers.

b) Das Konjugat kann auch auf folgende Weise hergestellt werden:

30 nMol des Antikörpers werden an einem makroporösen, stark sauren Kationenaustauscher gebunden, der zuvor mit einem 0,1 m-Natriumacetatpuffer (pH 5) äquilibriert wurde. Der Austauscher wird dann mit einer 0,03 m-Natriummetaperjodatlösung in Acetatpuffer gespült. Mit dem Auftauchen von Perjodat im Eluat wird der Durchfluß für 30 Minuten unterbrochen. Dann wird die Festphase mit Acetatpuffer gewaschen und im nächsten Schritt eine 0,03 M-Polyethyleniminpolyessigsäurepolyhydrazidlösung in Acetatpuffer aufgezogen. Nach dem Auftauchen des Komplexbildners im Eluat wird der Durchfluß für 2 Stunden unterbrochen. Anschließend eluiert man nicht gekoppelten Komplexbildner mit Acetatpuffer. Die Elution des Antikörperkonjugats von der Festphase wird mit Hilfe eines Kochsalz-Gradienten in Acetatpuffer vorgenommen. Die Isolierung des Konjugats erfolgt durch Gefriertrocknung der entsalzten Lösung.

c) Die Komplexierung des nach a) oder b) erhaltene Antikörperkonjugats erfolgt in folgender Weise:

20 mg des Konjugats werden in 4 ml eines 50 mMol Natriumacetat / 150 mMol Natriumchlorid-Puffers (pH 6,0) gelöst. Hierzu fügt man eine Lösung von 6.7 µMol Gadolinium(III)-chlorid in demselben Puffer und inkubiert 4 Stunden bei 2 °C. Nach Zugabe von 6,7 µMol Natriumcitrat läßt man 30 Minuten nachrühren und dialysiert erschöpfend gegen Natriumacetatpuffer. Durch Gefriertrocknung der Lösung erhält man als weißes Pulver ein Komplexkonjugat, das 85$\pm$3 Gadoliniumionen pro Antikörperkonjugat gebenden enthält.

Beispiel 8

Indiumkomplex des Konjugats aus Polyethyleniminpolyessigsäure und des monoklonalen Antikörpers MARG11

12 mg Polyethyleniminpolyessigsäure und 20 µl Triethylamin werden in 0,5 ml Acetonitril gelöst und mit 2 mg N-Hydroxysuccinimid und 2,5 mg Diisopropylcarbodiimid 3 Stunden gerührt.

Gleichzeitig bereitet man eine Lösung von 2 mg des monoklonalen Antikörpers MARG11 in 1 ml wäßrigen Puffer (0,1 M Natriumhydrogencarbonat und 0,2 M Ethylendiamintetraessigsäure pH7). Hierzu fügt man 100 µl der obigen Lösung des aktivierten Esters und läßt 1 Stunde unter gelegentlichem Schütteln stehen.

Nach Reinigung über einen Kationenaustauscher erfolgt die Komplexierung mit $^{111}InCl_3$. Nach Dialyse und Gefriertrocknung beträgt die spezifische Aktivität 560 m Ci/mg.

Beispiel 9

Konjugat des Gadolinium-Komplexes des Polyethyleniminpolyessigsäurepolyhydrazids mit Fab-Fragmenten des monoklonalen Antikörpers MARG11.

a) Herstellung von $F(ab')_2$-Fragmenten:

16 mg (100 nMol) des Antikörpers MARG2b8 (Subklasse IgG2b; Camon Laborservice GmbH, Wiesbaden) werden in 1 ml eines Gemischs von 0,1 m-Acetatpuffer (pH 4,5) und 0,1 M Kochsalzlösung gelöst und nach Zugabe von 0,3 mg Pepsin 20 Stunden bei 37 °C inkubiert. Nach Reinigung über Ultragel AcA 44 (Firma LKB) bei pH 7,0 und nach Gefriertrocknung erhält man 6,3 mg (63 % der Theorie) der gewünschten Fragmente.

b) Herstellung und Kopplung von Fab-Fragmenten:

15 mg (150 nMol) der nach a) erhaltenen Fragmente werden in 14,5 ml 0,1 M-Phosphatpuffer (pH 6,0) gelöst und mit 0,15 ml einer 0,1 M-Mercaptoethylaminlösung in 0,1 M-Phosphatpuffer (pH 6,0) unter Zusatz von 15 mMol Ethylendiamintetraessigsäure gelöst. Nach 2stündigem Inkubieren bei 37 °C trennt man unter

Argonschutz über eine Sephadex G 25-Säule ab. Eine Bestimmung der Sulfhydrylgruppen ergibt 238 nMol SH-Gruppen im Reaktionsansatz.

236 mg des in Beispiel 3 beschriebenen Gadolinium-Komplexes werden in 10 ml 0,1 m-Phosphatpuffer (pH 6,0) gelöst. Hierzu fügt man bei 10 °C 0,5 ml einer 0,1 M-Lösung von Sulfonsuccinimidyl-4-(4-maleimidophenyl)-butyrat ("Sulfo-SMPB", Firma Pierce Chemical) im gleichen Puffer und rührt 2 Stunden bei Raumtemperatur nach.

Anschließend vereinigt man die Lösung des Fragments mit der Lösung des aktivierten Komplexes und läßt über Nacht unter leichtem Schütteln reagieren. Die anschließende Aufarbeitung über einen Kationenaustauscher erfolgt in der in Beispiel 7 beschriebenen Verfahrensweise. Durch Gefriertrocknung der aufgereinigten Lösung erhält man 24,1 mg eines weißen Pulvers mit einem Gehalt von 8,89 Gew.% Gadolinium.

Beispiel 10

Gadolinium-Komplex der Polyethyleniminpolymethylenphosphonsäure

21,17 g Polyethyleniminpolymethylenphosphonsäure, hergestellt nach US 2599807, werden in 300 ml bidest. Wasser gelöst und mit 4,3 g Gadoliniumcarbonat (hydratisiert, 59 Gew % gd) unter Rühren 2 Stunden auf 80 °C erhitzt.

Nach Ultrafiltration und Gefriertrocknung erhält man 23.54 g weißes, voluminöses Material, das laut Elementaranalyse 10,29 Gew % Gadolinium enthält.

Beispiel 11

Biotinylierter Gadolinium-Komplex des Polyethyleniminpolyessigsäurepolyhydrazids

1,31 g des in Beispiel 3b beschriebenen Gd-Komplexes des Polyethylenimin-polyessigsäurepolyhydrazids werden in 20 ml Wasser mit 133 mg Sulfo-NHS-Biotin (Pierce Chemical Company) versetzt und durch Zugabe von $NaHCO_3$ bei pH 8-9 gehalten. Nach mehrstündigem Rühren bei Raumtemperatur verdünnt man mit Wasser und dialysiert, bis im Filtrat kein Biotin mehr nachweisbar ist. Nach Gefriertrocknung erhält man 1,08 g farblose Substanz. Biotin-Gehalt: 1,3 Mol%

Beispiel 12

Natrium-Salz der Polyethyleniminpolybernsteinsäure

Zu einer Lösung von 36,15 g Polyethylenimin (Polymin P) in 320 ml Methanol fügt man, gegebenenfalls unter vorsichtigem Erwärmen, eine Lösung von 181,75 g Maleinsäuredimethylester in 200 ml Methanol so zu, daß die Temperatur 40°C nicht übersteigt. Man rührt danach noch 4 Stunden bei 50-60°C und versetzt dann mit 53,8 g Natriumhydroxidplätzchen, wobei unter Rühren eine maximale Temperatur von 40°C eingehalten wird. Man gießt 200 ml Eiswasser zu und engt am Rotationsverdampfer ein. Der Rückstand wird in 500 ml 0,1 m Ammoniumacetat-Puffer gelöst und dialysiert. Nach Gefriertrocknen erhält man 116,39 g farbloser voluminöser Kristalle, die sich oberhalf 300°C unter Verfärbung zersetzen.

Gadolinium-Komplex:

Der Gadolinium-Komplex wird, wie für Beispiel 1a) beschrieben, hergestellt und enthält 12,49 Gew% Gadolinium

Beispiel 13

Gadoliniumkomplex des Polyethyleniminpolyessigsäurepolymethylesterpolyhydrazids

11,35 g des in Beispiel 2a beschriebenen Gadoliniumkomplexsalzes der Polyethyleniminpolyessigsäure werden in 250 ml wäßrigem Methanol mit 5,9 ml Dimethylsulfat versetzt, 6 Stunden bei Raumtemperatur gerührt und anschließend im Heizbad auf 60°C erwärmt. Danach läßt man auf Raumtemperatur abkühlen und gibt 0,5 ml Hydrazinhydrat zu und erhitzt anschließend noch 4 Stunden auf dem Dampfbad. Die so erhaltene Lösung wird dialysiert und gefriergetrocknet. Man erhält 11,5 g farblose Kristalle mit einem Gadoliniumgehalt von 10,6 Gew%. Die Bestimmung der Methylestergruppen ergibt eine Veresterung von 65% aller Carboxygruppen. Hydrazin (colorimetrisch nach Hydrolyse): 1,17 Mol%

**Beispiele für die Herstellung der Darreichungsformen**

Beispiel 14

Lösung vom Gadoliniumkomplex der Polylysinpolyessigsäure

5,98 g (entsprechend 4 mMol $Gd^{3+}$) des nach Beispiel 6 hergestellten Gadolinium-Komplexes werden in 800 ml Wasser pro injectione (p.i.) durch Zusatz von N-Methylglucamin bei pH 7,2 in Lösung gebracht. Nach Auffüllen mit Wasser p.i. auf 1000 ml wird die Lösung steril filtriert, in 10 ml Aliquots in Multivials abgefüllt und lyophilisiert. Der Rückstand entspricht jeweils einer Einzeldosis von 0,04 mMol $Gd^{3+}$/kg Körpergewicht.

Beispiel 15

Lösung des Natriumsalzes vom Indium-111-Komplex der Polyethyleniminpolyessigsäure

Eine Lösung von 100 µg einer Polyethyleniminpolyessigsäure mit einem mittleren Molekulargewicht von 100000 in 5 ml eines Gemischs aus einer 150 mmolaren Kochsalz- und einer 150 mmolaren Natriumacetatlösung (pH 5,8) wird mit einer Lösung von 5 mCi Indium-111-chlorid in 1 ml n-Salzsäure versetzt. Man bringt durch Zugabe von 1 n-Natronlauge auf pH 6 und dialysiert gegen Natriumacetatlösung, darauf gegen Kochsalzlösung. Dann bringt man durch Zugabe von 0,1 n-Natronlauge auf pH 7,2, füllt die steril filtrierte Lösung in Multivials ab und lyophilisiert sie. Der Rückstand wird in physiologischer Kochsalzlösung aufgenommen und stellt dann ein für die Radiodiagnostik geeignetes Präparat dar.

In analoger Weise erhält man aus 5 mg Polyethyleniminpolyessigsäure und einer Lösung von 500 mCi Yttrium-90-chlorid ein für die Radiotherapie geeignetes Präparat.

Beispiel 16

Lösung vom In-111-Komplex des Konjugats des Polyethyleniminpolyessigsäurehydrazids mit dem monoklonalen Antikörper MARG11

Eine Lösung von 200 µg des in Beispiel 7 beschriebenen Komplexbildners in 5 ml eines Gemischs aus einer 150 mmolaren Kochsalz- und einer 150 mmolaren Natriumacetatlösung (pH 5,8) wird mit einer auf pH 5 eingestellten Lösung von 5 m Ci Indium-111-chlorid in verdünnter Salzsäure versetzt. Die Aufarbeitung entsprechend Beispiel 11 liefert ein für die Radiodiagnostik geeignetes Präparat.

In analoger Weise erhält man aus 10 mg des in Beispiel 7 beschriebenen Komplexbildners und einer Lösung von 500 mCi Yttrium-9-chlorid (pH 5) ein für die Radiotherapie geeignetes Präparat.

Beispiel 17

Injektionslösung vom Gadoliniumkomplex des Konjugats des monoklonalen Antikörpers MARG 11 mit Polyethyleniminpolysäurepolyhydrazid

1,08 des unter Beispiel 7c) beschriebenen Antikörper-Gadolinium-Komplex-Konjugats werden in 80 ml Wasser p.i. gelöst, mit 50 mg des Calcium-Di-Natrium-Komplexes der DTPA versetzt und auf 100 ml aufgefüllt. Nach Sterilfiltration füllt man in 5 ml Multivials und lyophilisiert.

Durch Lösen in 5 ml physiologischer Kochsalzlösung erhält man ein Präparat für die parenterale Anwendung.

Beispiel 18

Injektionslösung des N-Methyl-D-Glucaminsalzes vom Gadolinium-Komplex der Polyethyleniminpolyessigsäure

127 g (= 50 mMol $Gd^{3+}$) des in Beispiel 2 c) beschriebenen N-Methyl-D-Glucamin-Salzes des Gd-Komplexes der Polyethyleniminpolyessigsäure werden in 800 ml Wasser p.i. gelöst, mit 1,2 g des Calcium-Di-Natriumsalzes der DTPA versetzt und auf 1 l mit Wasser p.i. aufgefüllt.

Die Lösung wird sterilfiltriert, in Portionen von je 5 ml in Multivials abgefüllt und lyophilisiert. Durch Lösen in 5 ml physiologischer Kochsalzlösung erhält man ein Präparat für die parenterale Anwendung.

Beispiel 19

Herstellung einer Lösung mit biotinyliertem Gadolinium-Komplex des Polyethyleniminpolyessigsäurepolyhydrazids zur in-vivo Applikation.

50 mg eines biotinylierten Gadoliniumkomplexes des Polyethyleniminpolyessigsäurepolyhydrazids (Biotin-Gehalt 1,3 Mol%) (Beispiel 11) werden in 10 ml physiologischer Kochsalzlösung gelöst.

Der pH wird mit Essigsäure auf pH 7,2 eingestellt und die Lösung mit einem 0,2 µm Filter steril filtriert. 0,2 ml dieser Lösung mit einem Gd-Gehalt von 0.9 µmol werden 48 Stunden nach Applikation eines tumorspezifischen monoklonalen Antikörper-Avidinkonjugates i.v. appliziert.

Die Durchführung einer NMR-diagnostischen Untersuchung unter Verwendung eines erfindungsgemäßen Polymer-Komplexes wird anhand der folgenden Beispiele näher erläutert:

Darstellung von Blutgefäßen in der Leber nach Applikation von 0,02 mmol Gadolinium als Gadolinium-Komplex der Polyethyleniminpolyessigsäure (Beispiel 1)

Eine Maus (NMRI, SPF Schering) wird mit Nembutal i.p. narkotisiert. Das Tier wird in einem 2 Tesla Kernspintomographen (General Electric) plaziert. Es werden im Bereich der Leber Aufnahmen (Transversalschnitt) in Spin-Echho-Sequenz mit $T_R$ = 400 msec und $T_E$ = 30 msec gemacht (Abb. 1). Dann werden 0,02 mmol Gadolinium in Form des Gadolinium-Komplexes der Polyethy-leniminpolyessigsäure (Beispiel 1), gelöst in 0,9% physiologischer Kochsalzlösung (200µl) i.v. appliziert. Zwei Minuten nach Applikation werden wiederum Aufnahmen in Spin-Echo-Sequenz ($T_R$ = 400 msec, $T_E$ = 30 msec) in der gleichen Schnittebene, wie oben beschrieben, gemacht (Abb. 2).

Abb. I

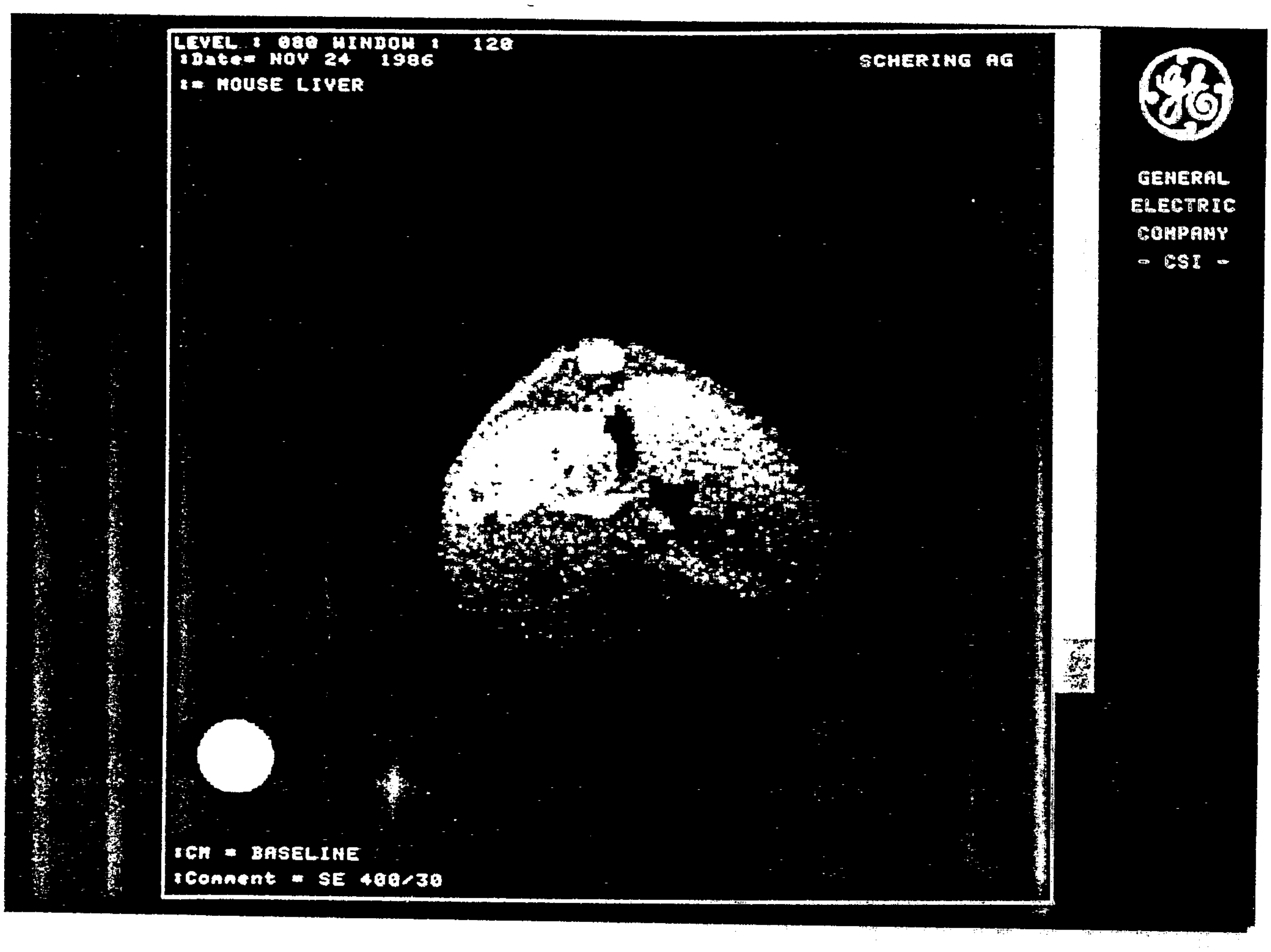


Kernspintomographische Aufnahme einer Maus im Bereich der Leber in Spin-Echo-Sequenz ($T_R$ = 400 msec, $T_E$ = 30 msec). Die senkrecht zur Bildebene verlaufenden großen Gefäße der Leber sind als dunkle Flecken zu erkennen. Links oben ist der Magen angeschnitten.

(Abb. II)

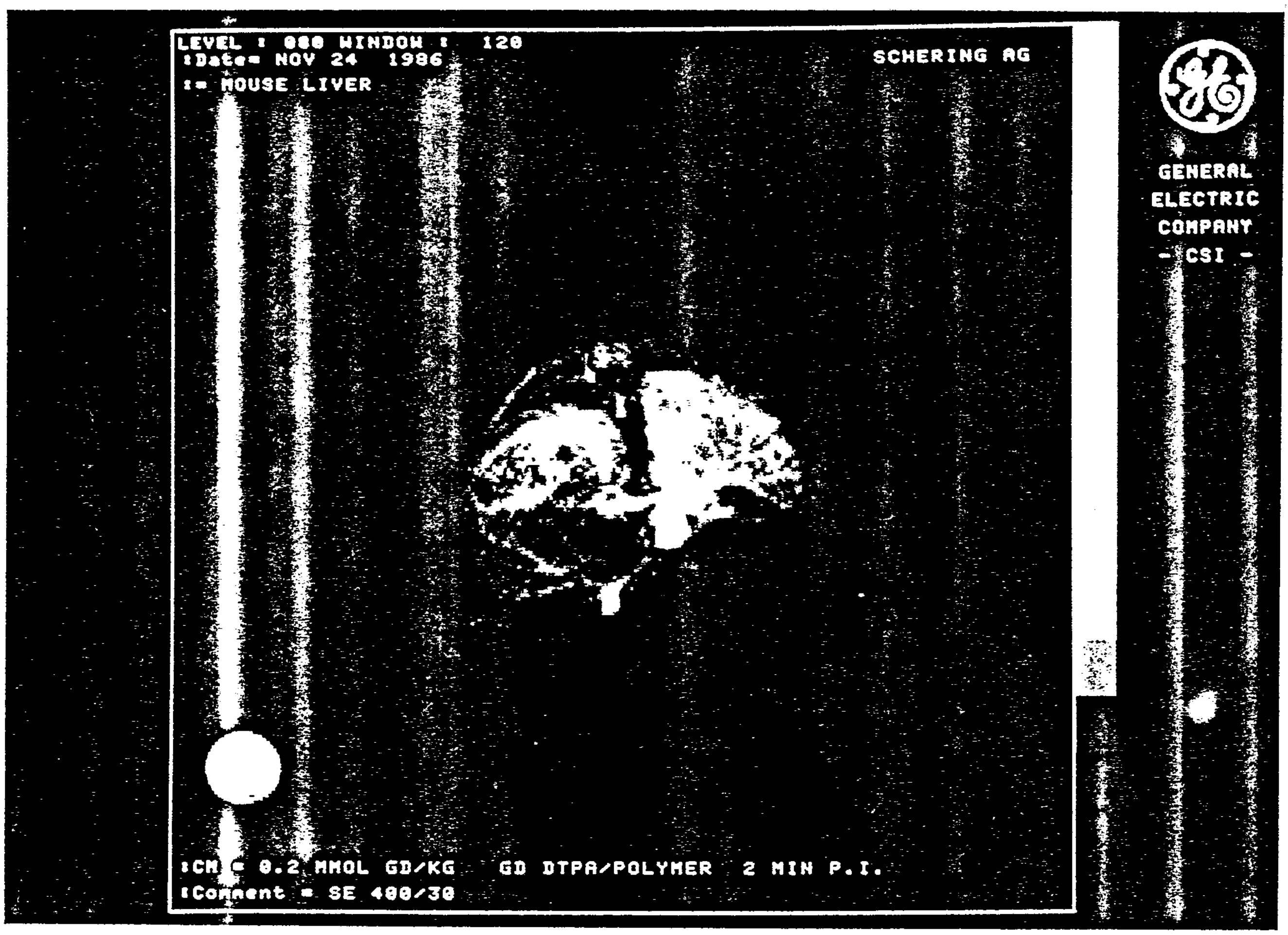


Kernspintomographische Aufnahme einer Maus im Bereich der Leber, 2 Minuten nach i.v. Applikation von 0,02 mmol Gadolinium als Gadolinium-Komplex der Polyethyleniminpolyessigsäure. Die senkrecht zur Bildebene verlaufenden Gefäße sind sehr deutlich durch den Anstieg in der Signalintensität erkennbar. Das gleiche gilt auch für die längs angeschnittenen kleineren Gefäße in der Leber.

## Patentansprüche

1.) Polymer-Komplexe bestehend aus einem Carbonsäure-, Phosphonsäure- und/oder Phenolgruppen enthaltenden Liganden mit alkylierten Amineinheiten, mindestens einem Ion eines Elements der Ordnungszahlen 21 - 29, 31, 32, 38, 39, 42 - 44, 49 oder 57 - 83 sowie gegebenenfalls Kationen anorganischer und/oder organischer Basen, Aminosäuren oder Aminosäureamide.

2.) Polymer-Komplexe gemäß Anspruch 1, dadurch gekennzeichnet, daß sie als Liganden eine Verbindung der allgemeinen Formel I

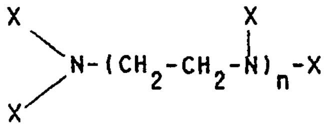

(I)

enthalten,
worin
n die ganzen Zahlen 7 bis 20.000,
X ein Wasserstoffatom, ein $CR^1R^2R^3$- oder ein $CR^1R^2$- U-Rest mit U in der Bedeutung eines

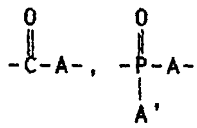 oder 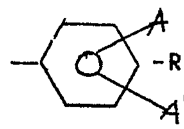 -Restes,

worin
A' für OH oder A und A" für H oder A' stehen, mit
A in der Bedeutung einer $(NH)_u$-$[B$-$(NH)_v]_w$-W- oder $OR^4$-Gruppe, worin
u und v für die Ziffern 0, 1 oder 2,
w für die Ziffern 0 oder 1,
$R^1$ für eine $(CH_2)_m$ Z-Gruppe,
$R^2$ für ein Wasserstoffatom oder eine $(CH_2)_l$ Z-Gruppe,
$R^3$ für ein Wasserstoffatom oder eine $(CH_2)_k$ Z-Gruppe und
$R^4$ für einen $C_1$-$C_6$-Alkyl- oder Benzylrest stehen,
wobei
m, l und k für die Ziffern 0 bis 10 und
Z für ein Wasserstoffatom, eine $C_1$-$C_{20}$-Alkyl-, eine -COOH-, $PO_3H_2$-oder

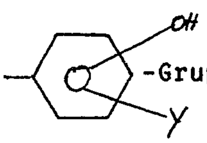 -Gruppe

stehen, worin Y ein Wasserstoffatom oder den Rest OH bedeutet,
B eine $C_0$-$C_{20}$-Alkylengruppe und
W ein Wasserstoffatom, ein Makromolekül, eine gegebenenfalls über eine geradkettige, verzweigte, gesättigte oder ungesättigte $C_1$-$C_{20}$-Alkylengruppe gebundene funktionelle Gruppe oder gebunden über diese funktionelle Gruppe ein Makro- oder Biomolekül, wobei die Alkylengruppe gegebenenfalls Imino-, Phenylenoxy-, Phenylenimino-, Amid-, Estergruppe(n), Sauerstoff-, Schwefel- und/oder Stickstoff-Atom(e) enthalten und gegebenenfalls durch Hydroxy-, Mercapto- und/oder Aminogruppe(n) substituiert ist, bedeuten,
wobei die Substituenten X in den einzelnen Gliedern und an den Enden der Verbindung sowie die Substituenten Z in $R^1$, $R^2$ und $R^3$ gleich oder verschieden sein können, mit den Maßgaben, daß nicht alle X gleichzeitig Wasserstoff bedeuten sollen, daß zumindest ein Z nicht Wasserstoff oder $C_1$-$C_{20}$-Alkyl bedeutet, daß im Falle von m, l und k = 0 nicht alle Substituenten Z für -COOH, $-PO_3H_2$ oder

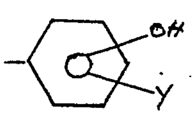

stehen und daß gewünschtenfalls ein Teil der -COOH-, $PO_3H_2$- oder

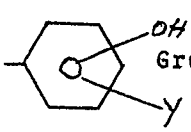 Gruppen

als Ester oder Amide vorliegt.

3.) Polymer-Komplexe gemäß Anspruch 2, dadurch gekennzeichnet, daß sie zwischen 1 und n/10 $CR^1R^2$ -U-Rest(e),

worin n, $R^1$, $R^2$ und U die oben genannte Bedeutung haben, enthalten.

4.) Polymer-Komplexe gemäß Anspruch 1, dadurch gekennzeichnet, daß sie als Liganden

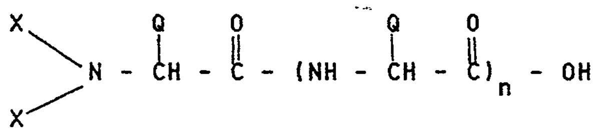 (II),

worin

Q den Rest einer, die Gruppe(n) NX und/oder $NX_2$ enthaltenden natürlichen Aminosäure bedeutet und n und X die in Anspruch 2 genannten Bedeutungen haben,

enthalten.

5.) Polymer-Komplexe gemäß Anspruch 1, dadurch gekennzeichnet, daß sie mindestens ein Ion eines Elements der Ordnungszahlen 21 - 29, 42, 44 oder 57 -83 oder mindestens ein Radionuklid eines Elements der Ordunungszahlen 27, 29, 31, 32, 38, 43, 49, 62, 64, 70 oder 77 enthalten.

6.) Polymer-Komplexe gemäß Anspruch 2 bis 4, dadurch gekennzeichnet, daß die funktionelle Gruppe W

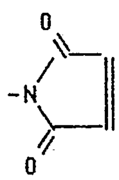 , 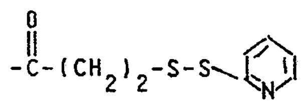 , 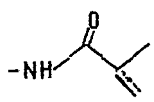 , 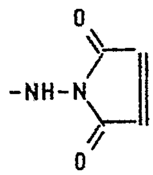 ,

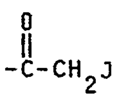, $-(CH_2)_3J$, $-(CH_2)_3SH$, $-(CH_2)_3NH_2$, $-C_6H_4SCN$,

bedeutet.

7.) Polymer-Komplexe gemäß Anspruch 2 bis 4, dadurch gekennzeichnet, daß das in W enthaltene Makromolekül ein Antikörper, ein Antikörper-Fragment, ein Avidin-Biotin-Antikörper oder Avidin-Biotin-Antikörper-Fragment ist.

8.) Pharmazeutische Mittel enthaltend mindestens einen Polymer-Komplex nach Anspruch 1 bis 7, gegebenenfalls mit den in der Galenik üblichen Zusätzen.

9.) Verwendung mindestens eines Polymer-Komplexes gemäß Anspruch 5 für die Herstellung von Mitteln für die NMR-, Röntgen-, Ultraschall-, Radio-Diagnostik oder Radiotherapie.

10.) Verwendung mindestens eines Polymer-Komplexes der allgemeinen Formeln I′ und II′, worin W für ein Wasserstoffatom oder eine funktionelle Gruppe steht, gemäß Anspruch 2 bis 4 als Hapten zur Herstellung von Antikörpern.

11.) Verfahren zur Herstellung von Polymer-Komplexen gemäß den Ansprüchen 1 bis 7, dadurch gekennzeichnet, daß man einen in an sich bekannter Weise durch N-Alkylierung erhaltenen, Carbonsäure-, Phosphonsäure- und/oder Phenolgruppen enthaltenden Liganden in an sich bekannter Weise

a) mit mindestens einem Metalloxid oder Metallsalz eines Elements der Ordnungszahlen 21 - 29, 31, 32, 38, 39, 42 - 44, 49 oder 57 - 83 umsetzt, oder

b) mit mindestens einem Metalloxid oder Metallsalz eines Elements der Ordnungszahlen 21 - 29, 31, 32, 38, 39, 42 - 44, 49 oder 57 - 83 umsetzt, mindestens ein Teil der nicht zur Komplexierung benötigten Carbonsäure-Phosphonsäure- oder Phenolgruppen des so erhaltenen Metallkomplexes in die Gruppe(n) $CR^1R^2$-U′ mit

U′ in der Bedeutung eines

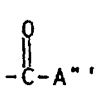, 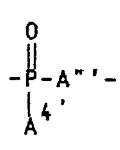 oder 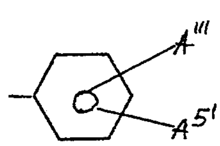

Restes,

worin

$A^{4'}$ für OH oder A‴, und $A^{5'}$ für H oder $A^{4'}$stehen,

mit

A‴ in der Bedeutung einer $(NH)_u$-$[B$-$(NH)_v]_w$-W′ oder $OR^4$-Gruppe

worin
u und v für die Ziffern 0, 1 oder 2,
w für die Ziffern 0 oder 1,
$R^1$ für eine $(CH_2)_m$ Z-Gruppe
$R^2$ für ein Wasserstoffatom oder eine $(CH_2)_l$ Z-Gruppe mit m und l in der Bedeutung der Ziffern 0 bis 10 und Z in der Bedeutung für ein Wasserstoffatom, eine $C_1$-$C_{20}$-Alkylgruppe, eine COOH-, $PO_3H_2$ oder

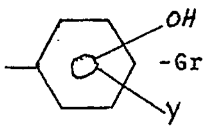
-Gruppe,

worin Y ein Wasserstoffatom oder den Rest OH bedeutet,
$R^4$ für einen $C_1$-$C_{20}$-Alkylrest oder Benzylrest,
B für ein $C_0$-$C_{20}$-Alkylengruppe und
W' für ein Wasserstoffatom oder eine gegebenenfalls über eine geradkettiige, verzweigte, gesättigte oder ungesättigte $C_1$-$C_{20}$-Alkylengruppe gebundene funktionelle Gruppe, wobei die Alkylengruppe gegebenenfalls Imino, Phenylenoxy-, Phenylenimino-, Amid-, Estergruppe(n), Sauerstoff-, Schwefel-und/oder Stickstoff-Atom(e) enthält und gegebenenfalls durch Hydroxy-, Mercapto- und/oder Aminogruppe(n) substituiert ist,
stehen,
umwandelt, wobei die $CR^1R^2U'$-Gruppen gleich oder verschieden sein können, und anschließend, falls gewünscht, direkt oder über in dem oben genannten Rest enthaltene funktionelle Gruppe an ein Makro- oder Bio-molekül bindet,
oder
c) in ein mindestens eine $CR^1R^2$- U'-Gruppe enthaltenes Derivat überführt, wobei $R^1$, $R^2$ und U' die obengenannten Bedeutungen haben, dieses anschließend mit mindestens einem Metalloxid oder Metallsalz eines Elements der Ordnungszahlen 21-29, 31, 32, 38, 39, 42-44, 49 oder 57-83 umsetzt und da nach,falls gewünscht, direkt oder über die in dem oben genannten Rest enthaltene funktionelle Gruppe an ein Makro- oder Biomolekül bindet, oder
d) in ein mindestens eine $CR^1R^2$-U'-Gruppe enthaltenes Derivat überführt, wobei $R^1$, $R^2$ und U' die oben genannten Bedeutungen haben, dieses, falls W ein Makro- oder Biomolekül enthalten soll, direkt oder über die in dem oben genannten Rest enthaltene funktionelle Gruppe an ein Makromolekül bindet und anschließend mit mindestens einem Metalloxid oder Metallsalz eines Elements der Ordnugngszahlen 21 - 29, 31, 32, 38, 39, 42 44, 49 oder 57 - 83 umsetzt
und gegebenenfalls anschließend in den nach a), b), c) oder d) erhaltenen Polymer-Komplexen noch vorhandene azide Wasserstoffatome durch Kationen anorganischer und/oder organischer Basen, Aminosäuren oder Aminosäureamide substituiert.

12.) Verfahren zur Herstellung von Polymer-Komplexen gemäß Anspruch 14, dadurch gekennzeichnet, daß man die Kopplung des Makro-oder Biomoleküls an den funktionalisierten Polymer-Komplex oder Liganden sowie, im Falle der Kopplung an den Liganden, die nachfolgende Komplexierung mit dem/den gewünschten Metallionen an einer stationären Phase durchführt.

13.) Verfahren zur Herstellung der pharmazeutischen Mittel gemäß Anspruch 10, dadurch gekennzeichnet, daß man den in Wasser oder physiologischer Salzlösung gelösten oder suspendierten Polymer-Komplex, gegebenenfalls mit den in der Galenik üblichen Zusätzen, in eine für die enterale oder parenterale Applikation geeignete Form bringt.

0277088

Abb. I

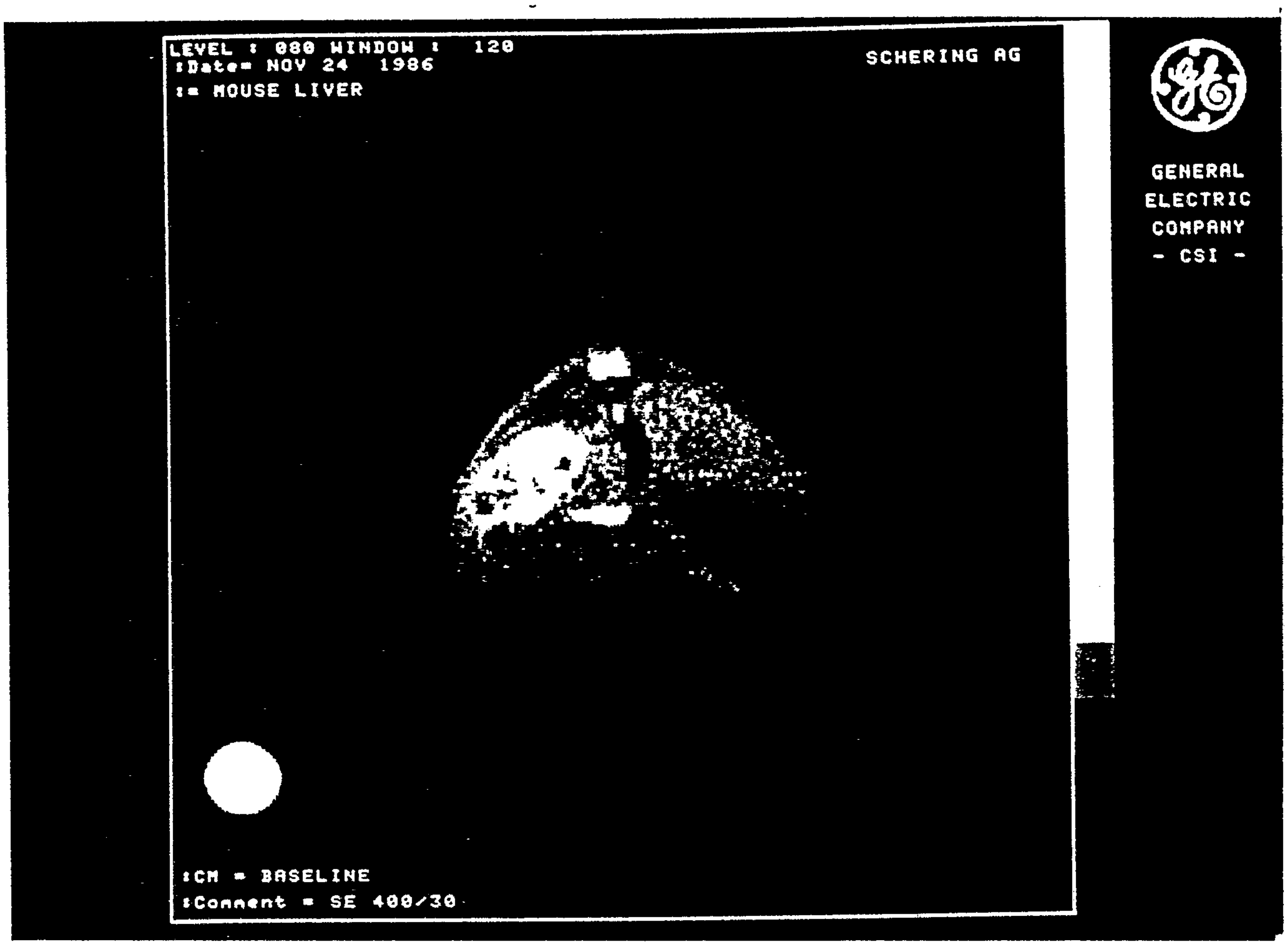


Kernspintomographische Aufnahme einer Maus im Bereich der Leber in Spin-Echo-Sequenz ($T_R$ = 400 msec, $T_E$ = 30 msec). Die senkrecht zur Bildebene verlaufenden großen Gefäße der Leber sind als dunkle Flecken zu erkennen. Links oben ist der Magen angeschnitten.

0277088

(Abb. II)

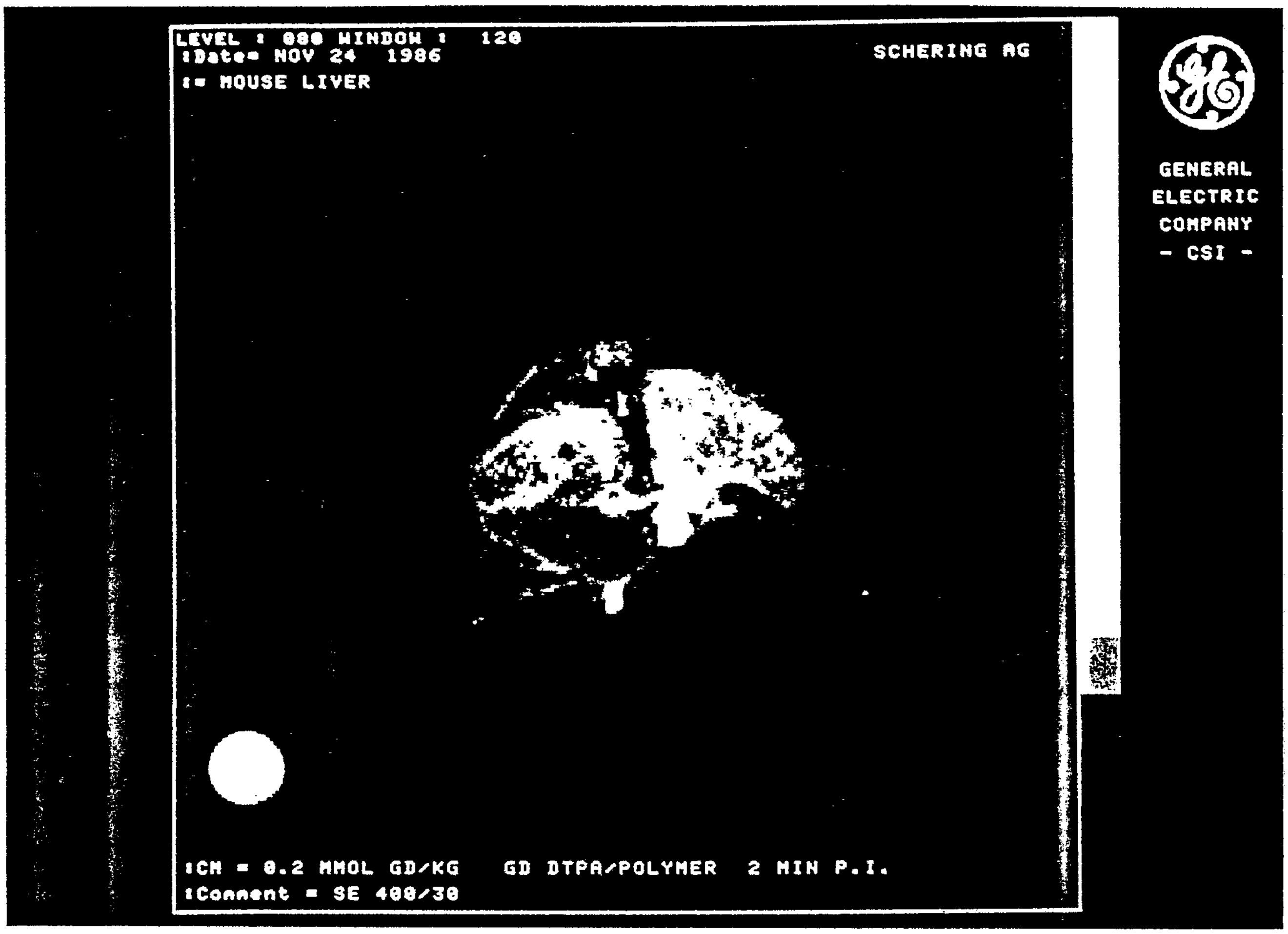


Kernspintomographische Aufnahme einer Maus im Bereich der Leber, 2 Minuten nach i.v. Applikation von 0,02 mmol Gadolinium als Gadolinium-Komplex der Polyethyleniminpolyessigsäure. Die senkrecht zur Bildebene verlaufenden Gefäße sind sehr deutlich durch den Anstieg in der Signalintensität erkennbar. Das gleiche gilt auch für die längs angeschnittenen kleineren Gefäße in der Leber.